# EUROPEAN PATENT APPLICATION

(11) **EP 4 242 224 A1**
(43) Date of publication of application: **13.09.2023**
(21) Application number: 21889638.9
(22) Date of filing: 05.11.2021
(51) Int. Cl.: C07K 16/10, A61P 31/14, C12N 15/10, A61K 39/00

(54) **ANTIBODY SPECIFIC TO CORONAVIRUS SPIKE PROTEIN AND USE THEREOF**

(30) Priority: 06.11.2020 KR 20200148064; 01.04.2021 KR 20210042796
(71) Applicant: Green Cross Corporation, Yongin-si, Gyeonggi-do 16924 (KR); Mogam Institute for Biomedical Research, Yongin-si, Gyeonggi-do 16924 (KR)
(72) Inventor: KIM, Dong-Sik, Yongin-si, Gyeonggi-do 16924 (KR); LEE, Sua, Yongin-si, Gyeonggi-do 16924 (KR); JANG, Shin A, Yongin-si, Gyeonggi-do 16924 (KR); KANG, Jihoon, Yongin-si, Gyeonggi-do 16924 (KR); CHO, KI Joon, Yongin-si, Gyeonggi-do 16924 (KR); PARK, Soo Bin, Yongin-si, Gyeonggi-do 16924 (KR); HAN, Young Woo, Yongin-si, Gyeonggi-do 16924 (KR); NAM, Hyemi, Yongin-si, Gyeonggi-do 16924 (KR); OH, Mi Young, Yongin-si, Gyeonggi-do 16924 (KR); LEE, Jee Boong, Yongin-si, Gyeonggi-do 16924 (KR); RYU, Jihye, Yongin-si, Gyeonggi-do 16924 (KR); KIM, Mun Kyung, Yongin-si, Gyeonggi-do 16924 (KR); LEE, Jeewon, Yongin-si, Gyeonggi-do 16924 (KR)
(74) Representative: Icosa
(86) International application number: PCT/KR2021/016073
(87) International publication number: WO 2022/098173

(57) **Abstract**

Provided are a coronavirus spike (S) protein-specific antibody and use thereof.

## Description

### BACKGROUND OF THE INVENTION

### 1. Field of the Invention

The present invention relates to a coronavirus spike (S) protein-specific antibody and use thereof.

### 2. Description of the Related Art

Coronavirus was first found in chickens in 1937, and then also in humans in 1965 through animals such as dogs, pigs, birds, *etc.* It was given this name because it resembles the corona phenomenon, in which the sun's photosphere is covered by the moon during a total solar eclipse, and the corona glows with a white light around the sun.

Coronavirus is known to cause mainly pneumonia and enteritis in humans and animals, and occasionally nervous system infections and hepatitis. Coronavirus is a positive-sense RNA virus with a size of about 100 nm to about 120 nm, belonging to the family Coronaviridae and having a spherical outer membrane. Coronavirus consists of a total of five structural proteins: the outermost spike protein (S), hemagglutinin-esterase (HE) protein, transmembrane (M) protein, small membrane (E) protein, and nucleocapsid (N) protein. Among them, the spike protein is a protein that acts as a ligand binding to the cell receptor and induces fusion between the host cell and the virus, and is known to mutate the most frequently.

Until now, coronavirus had been recognized as a pathogen that rarely infects humans and mainly infects animals such as dogs, pigs, cattle, *etc.* Even when coronavirus infects humans, it causes, as one of many viruses that cause respiratory symptoms, simple colds or intestinal diseases such as diarrhea, *etc*., which are not very dangerous to children. However, since new strains (variants) of coronavirus have been identified as the causative agents of severe acute respiratory syndrome (SARS), which has caused hundreds of deaths and thousands of patients worldwide, Middle East respiratory syndrome (MERS), and coronavirus disease 2019 (COVID-19), they have gradually begun to receive attention.

COVID-19 was first identified in Wuhan, the capital of Hubei province, China at the end of 2019, and has spread worldwide, resulting in a progressive pandemic, and is an infectious disease caused by SARS-coronavirus-2. As of May 7, 2020, more than 3.75 million cases have been reported in 187 countries, the death toll has reached 263,000, and 1.24 million have recovered. Common symptoms are fever, cough, fatigue, shortness of breath, and loss of smell and taste. In most cases, symptoms are mild, but some progress to viral pneumonia, multi-organ failure, and cytokine storm. The time from the onset of symptoms to the onset of the diseases is generally about 5 days, but may be between 2 days and 14 days. The virus spreads from person to person mainly during close contact, sometimes through droplets from coughing, sneezing, and talking. It is most contagious during the first 3 days after the onset of symptoms, and can be transmitted before the onset of symptoms or even at the end of the disease. The standard method of diagnosing COVID-19 uses real-time reverse transcription polymerase chain reaction (rRT-PCR) from a nasopharyngeal swab.

SARS-coronavirus-2 is known as a variant coronavirus belonging to the genus *Betacoronavirus*, and is believed to originate from bats, which are the primary carriers of various coronaviruses (Antiviral Res., 101 :45-56), and its exact infection route has not yet been fully elucidated.

On the other hand, since antiviral agents developed so far exhibit severe side effects, great care is required in their application. These therapeutic agents are not effective, and side effects are also appearing. In addition, there have been no new therapeutic agents for coronavirus with excellent infection suppression effect and low toxicity for the prevention and treatment of the occurrence of SARS-coronavirus-2, and the need for development is increasing.

The present inventors have made efforts to develop a novel therapeutic agent for SARS-coronavirus-2, and as a result, they found that a novel antibody specific to Receptor Binding Domain (RBD) of SARS-CoV-2 S protein has a therapeutic effect on coronaviruses, particularly SARS-CoV-2, thereby completing the present invention.

### SUMMARY OF THE INVENTION

An object of the present invention is to provide an antibody specifically binding to a coronavirus spike protein or an antigen binding fragment thereof.

Another object of the present invention is to provide a polynucleotide encoding the antibody or the antigen binding fragment, an expression vector including the polynucleotide, and a transformant including the expression vector.

Still another object of the present invention is to provide a method of preparing the antibody or the antigen binding fragment.

Still another object of the present invention is to provide a composition for diagnosing coronavirus infection, the composition including the antibody or the antigen binding fragment, and a kit including the composition.

Still another object of the present invention is to provide a method of providing information for diagnosing coronavirus infection, the method including the step of detecting coronaviruses present in a biological sample using the antibody or the antigen binding fragment.

Still another object of the present invention is to provide a pharmaceutical composition for preventing or treating coronavirus, the pharmaceutical composition including the antibody or the antigen binding fragment.

Still another object of the present invention is to provide a method of treating coronavirus using the antibody or the antigen binding fragment.

### BRIEF DESCRIPTION OF THE DRAWINGS

FIG. 1 shows the size and purity of antigens purified, which were confirmed through SDS-PAGE.
FIG. 2 shows the size and purity of anti-SARS-CoV-2 antibodies, which were confirmed through SDS-PAGE, after expression and purification of the anti-SARS-CoV-2 antibodies obtained through library screening. Specifically, the whole sizes (approximately 150 kDa) of the antibodies were confirmed through Non-reduced PAGE, and the sizes of heavy chain (approximately 50 kDa) and light chain (approximately 25 kDa) of the antibodies were confirmed through Reduced PAGE.
FIG. 3 shows the results of performing sandwich ELISA to select antigen-specific anti-COVID-19 antibodies, through which antibodies specifically binding to the antigen were selected.
FIG. 4 shows the experimental results of examining whether or not the selected anti-SARS-CoV-2 antibodies also bind to S (S1 S2) protein of other *Betacoronavirus*, SARS-CoV and MERS-CoV.
FIG. 5 shows the results of SDS-PAGE for confirming the IgG expression and purification of humanized clones. Specifically, the whole sizes (approximately 150 kDa) of the antibodies were confirmed through Non-reduced PAGE, and the sizes of heavy chain (approximately 50 kDa) and light chain (approximately 25 kDa) of the antibodies were confirmed through Reduced PAGE.
FIG. 6 shows the results of performing sandwich ELISA to select antigen-specific anti-COVID-19 humanized antibodies. Specifically, the results confirmed that all of the humanized clones maintained the same binding affinity as the existing.
FIG. 7 shows the results of comparing the binding affinity of the humanized antibodies for the SARS-CoV-2 mutant antigen. Specifically, both of M4-4 and M5-4 clones maintained the binding affinity for the mutant antigens at a higher level than the wild type.
FIG. 8A shows the results of kinetics among the binding properties of antibodies against anti-SARS-CoV-2 antigen. Specifically, a high dissociation constant value of ten-fold or more was measured in the humanized antibody M4-4, as compared to the existing antibody M4.
FIG. 8B shows the results of assessing the binding affinity for alpha, beta, gamma mutants among the binding properties of antibodies against anti-SARA-CoV-2 antigen. Specifically, M4-4 was confirmed to have very strong binding affinity for all mutants.
FIG. 9A shows the results of flow cytometry for examining ACE2 expression of ACE2-HEK293 cells.
FIG. 9B shows the results of measuring SARS-Cov-2 pseudovirus-neutralizing potency of antibody candidates using SARS-CoV-2 pseudovirus and ACE2-HEK293 cells.
FIG. 10A shows the results of flow cytometry for examining spike protein expression of HT1080-S cells.
FIG. 10B shows the results of measuring ADCC efficacy of antibody candidates using ADCC Effector cells and HT1080-S cells.
FIG. 10C shows the results of measuring ADCP efficacy of antibody candidates using FcγRIIa-H Effector cells and HT1080-S cells.
FIG. 11 shows neutralizing activity of anti-SARS-CoV-2 antibody against authentic, wild-type antigen, alpha, beta, and gamma mutant antigens. Specifically, in all cases, M4-4 antibody showed neutralizing potency at the level equivalent or higher than that of the comparative antibody.
FIG. 12 shows the results of epitope binning of M4-4 antibody. Specifically, the results suggest that M4-4 binds to a wide epitope by competing 100% with REGN10987, S309, and ACE2 receptor.
FIG. 13 shows the results of simulation docking for predicting the epitope of M4-4 antibody. Specifically, the results were consistent with epitope prediction data.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present invention pertains.

One aspect of the present invention provides an antibody specifically binding to a coronavirus spike (S) protein or an antigen binding fragment thereof.

As used herein, the term "coronavirus" refers to a positive-sense single-stranded RNA virus (ssRNA) with a size of about 80 nm to about 220 nm, belonging to the family Coronaviridae and having a spherical outer membrane. Coronavirus is known to include a total of five structural proteins: the outermost spike protein (S), hemagglutinin-esterase (HE) protein, membrane (M) protein, envelope (E) protein, and nucleocapsid (NP) protein (Lai and Homes, 2001. Fields Virology).

In one embodiment, the coronavirus may be SARS-CoV, SARS-CoV-2, or MERS-CoV, specifically SARS-CoV-2, but is not limited thereto. The coronavirus spike (S) protein may be spike1 (S1) protein, in which some domains of the spike (S) protein of SARS-CoV-2 are cleaved, but any coronavirus is included without limitation, as long as it is able to specifically bind to the antibody of the present invention or the antigen binding fragment thereof.

As used herein, the term "SARS-CoV-2 (SARS-coronavirus-2)" refers to a virus that causes coronavirus infection-19 (COVID-19) which is a respiratory disease.

SARS-CoV-2 is a positive-sense single-stranded RNA virus. SARS-CoV-2 is taxonomically a strain of SARS-CoV, has zoonotic origins, and is considered to have close genetic similarities to bat coronaviruses. The SARS-CoV-2 is mainly transmitted through close contact between people and/or through droplets generated during coughing or sneezing, and it is known that the outer spike (S) protein of the virus mainly binds to a receptor angiotensin converting enzyme 2 (ACE2) present on the surface of human cells to enter the human cells.

SARS-CoV-2 may include RNA-dependent RNA polymerase, spike (S), membrane (M), envelope (E), and nucleocapsid (NP) proteins and/or genes encoding the proteins. Among the SARS-CoV-2 structural proteins, the spike (S) protein exists on the surface of virus particles and is involved in host invasion, and is divided into S1 and S2. S1 protein interacts with receptors of host cells, and the receptor binding domain (RBD) present in S1 protein is known as a key site that binds to ACE2 receptors present on the surface of host cells. S2 protein is involved in fusion with cell membranes of host cells. The structure is described in detail in Cascella, M., Rajnik, M., Cuomo, A., Dulebohn, S. C., & Di Napoli, R. (2020). Features, Evaluation and Treatment Coronavirus (COVID-19), in StatPearls, Treasure Island (FL).

Specifically, the antibody or fragment thereof provided in the present invention may specifically bind to spike1 (S1) protein, in which some domains of the spike (S) protein of SARS-CoV-2 are cleaved. Specifically, the antibody specifically binding to the coronavirus spike protein may be a mouse antibody, a chimeric antibody, or a humanized antibody.

For example, an antigenic determinant (epitope) recognized by the antibody or fragment thereof provided in the present invention may be a part of the connector domain (CD) present on the spike1 (S1) protein of SARS-CoV-2.

As used herein, the term "antibody" refers to a protein molecule that serves as a ligand specifically recognizing an antigen, including an immunoglobulin molecule immunologically reactive to a specific antigen, and encompasses all of polyclonal antibodies, monoclonal antibodies, whole antibodies, and antibody fragments. Further, the term encompasses chimeric antibodies (*e.g*., humanized murine antibodies) and bivalent or bispecific molecules (*e.g*., bispecific antibodies), diabodies, triabodies, and tetrabodies. The term includes also single chain antibodies retaining the FcRn (neonatal Fc receptor) binding function, SCAP, derivatives of antibody constant region, and artificial antibodies based on protein scaffold. A whole antibody has a structure consisting of two full-length light chains (LC) and two full-length heavy chains (HC), each light chain linked to heavy chain via disulfide bonds. The whole antibody includes IgA, IgD, IgE, IgM, and IgG, and IgG includes subtypes of IgG1, IgG2, IgG3, and IgG4. In one embodiment, the antibody provided in the present invention may be an IgG antibody.

As used herein, the terms "fragment", "binding fragment of a polypeptide", and "antibody fragment" refer to any fragment of the antibody of the present invention, which retains the antigen binding activity of the antibody, and they are used interchangeably. Exemplary antibody fragments include, but are not limited to, single chain antibodies, Fd, Fab, Fab', F(ab')₂, dsFv, or scFv. Fd refers to a portion of the heavy chain which is included in the Fab fragment. Fab has a structure composed of variable regions of light and heavy chains, the constant region (framework region, FR) of light chain, and the first constant region (CH1 domain) of heavy chain, with one antigen binding site. Fab' is different from Fab in that it has a hinge region including one or more cysteine residues at the C-terminus of the heavy chain CH1 domain. F(ab')₂ antibody is produced by a disulfide bond between the cysteine residues of the hinge region of Fab'. Fv (variable fragment) refers to the smallest antibody fragment composed of only the heavy chain variable region and the light chain variable region. Disulfide Fv (dsFv) is formed by linking the heavy chain variable region and the light chain variable region via a disulfide bond. Single chain Fv (scFV) is generally formed by covalently linking the heavy chain variable region and the light chain variable region via a peptide linker. These antibody fragments may be obtained using proteases (*e.g*., digestion of a whole antibody with papain or pepsin affords Fab or F(ab')₂, respectively), and for example, they may be constructed using genetic recombination technology.

As used herein, the term "antigenic determinant" is also called "epitope", and refers to a region or a site on an antigen, to which an antibody or antibody fragment specifically binds.

Further, the antibody or antigen binding fragment thereof may competitively bind to an epitope derived from the coronavirus spike to which casirivimab (REGN10933), imdevimab (REGN10987), and/or S309 bind, but is not limited thereto.

In one embodiment, the antibody provided in the present invention may be a monoclonal antibody.

As used herein, the term "monoclonal antibody" refers to an antibody molecule with a uniform molecular composition, obtained from a substantially identical population of antibodies, and such a monoclonal antibody exhibits single binding specificity and affinity for a particular epitope.

Typically, an immunoglobulin has heavy and light chains, each heavy chain and light chain includes constant and variable regions (also known as "domains"). The variable regions of the light chain and the heavy chain include three hypervariable regions, also called complementarity-determining regions (hereinafter referred to as "CDRs"), and four framework regions. Generally, CDRs function to bind to an epitope of an antigen. CDRs on each chain are sequentially named CDR1, CDR2, and CDR3, starting from the N-terminus. They are discriminated by the chain on which specific CDRs are positioned.

Generally, an immunoglobulin has heavy and light chains, and each heavy chain and light chain includes constant and variable regions. The variable regions of the light and heavy chains include three hypervariable regions, also called complementarity-determining regions (hereinafter referred to as "CDRs"), and four framework regions (hereinafter referred to as "FRs"). CDRs on each chain function to bind to an epitope of an antigen, and typically are sequentially named CDR1, CDR2, and CDR3, starting from the N-terminus. Further, the FRs on each chain may be named sequentially FR1, FR2, FR3, and FR4, starting from the N-terminus.

In the present invention, the variable region of the heavy chain may be named "V_{H}"; the variable region of the light chain may be named "V_{L}"; CDRs of the heavy chain may be named "V_{H}-CDR1", "V_{H}-CDR2", and "V_{H}-CDR3"; CDRs of the light chain may be named "V_{L}-CDR1", "V_{L}-CDR2", and "V_{L}-CDR3"; FRs of the heavy chain may be named "V_{H}-FR1 ", "V_{H}-FR2", "V_{H}-FR3", and "V_{H}-FR4"; and FRs of the light chain may be named "V_{L}-FR1 ", "V_{L}-FR2", "V_{L}-FR3", and "V_{L}-FR4".

In one embodiment, the antibody of the present invention specifically binding to the coronavirus spike protein or the antigen binding fragment thereof may include any one or more heavy chain variable regions selected from the group including any one sequence of SEQ ID NOS: 5 to 45, SEQ ID NOS: 87 to 91, and SEQ ID NOS: 97 to 115; and any one or more light chain variable regions selected from the group including any one sequence of SEQ ID NOS: 46 to 86, SEQ ID NOS: 92 to 96, and SEQ ID NOS: 116 to 131. Specifically, it may include any one of a heavy chain variable region of SEQ ID NO: 5 and a light chain variable region of SEQ ID NO: 46; a heavy chain variable region of SEQ ID NO: 6 and a light chain variable region of SEQ ID NO: 47; a heavy chain variable region of SEQ ID NO: 7 and a light chain variable region of SEQ ID NO: 48; a heavy chain variable region of SEQ ID NO: 8 and a light chain variable region of SEQ ID NO: 49; a heavy chain variable region of SEQ ID NO: 9 and a light chain variable region of SEQ ID NO: 50; a heavy chain variable region of SEQ ID NO: 10 and a light chain variable region of SEQ ID NO: 51; a heavy chain variable region of SEQ ID NO: 11 and a light chain variable region of SEQ ID NO: 52; a heavy chain variable region of SEQ ID NO: 12 and a light chain variable region of SEQ ID NO: 53; a heavy chain variable region of SEQ ID NO: 13 and a light chain variable region of SEQ ID NO: 54; a heavy chain variable region of SEQ ID NO: 14 and a light chain variable region of SEQ ID NO: 55; a heavy chain variable region of SEQ ID NO: 15 and a light chain variable region of SEQ ID NO: 56; a heavy chain variable region of SEQ ID NO: 16 and a light chain variable region of SEQ ID NO: 57; a heavy chain variable region of SEQ ID NO: 17 and a light chain variable region of SEQ ID NO: 58; a heavy chain variable region of SEQ ID NO: 18 and a light chain variable region of SEQ ID NO: 59; a heavy chain variable region of SEQ ID NO: 19 and a light chain variable region of SEQ ID NO: 60; a heavy chain variable region of SEQ ID NO: 20 and a light chain variable region of SEQ ID NO: 61; a heavy chain variable region of SEQ ID NO: 21 and a light chain variable region of SEQ ID NO: 62; a heavy chain variable region of SEQ ID NO: 22 and a light chain variable region of SEQ ID NO: 63; a heavy chain variable region of SEQ ID NO: 23 and a light chain variable region of SEQ ID NO: 64; a heavy chain variable region of SEQ ID NO: 24 and a light chain variable region of SEQ ID NO: 65; a heavy chain variable region of SEQ ID NO: 25 and a light chain variable region of SEQ ID NO: 66; a heavy chain variable region of SEQ ID NO: 26 and a light chain variable region of SEQ ID NO: 67; a heavy chain variable region of SEQ ID NO: 27 and a light chain variable region of SEQ ID NO: 68; a heavy chain variable region of SEQ ID NO: 28 and a light chain variable region of SEQ ID NO: 69; a heavy chain variable region of SEQ ID NO: 29 and a light chain variable region of SEQ ID NO: 70; a heavy chain variable region of SEQ ID NO: 30 and a light chain variable region of SEQ ID NO: 71; a heavy chain variable region of SEQ ID NO: 31 and a light chain variable region of SEQ ID NO: 72; a heavy chain variable region of SEQ ID NO: 32 and a light chain variable region of SEQ ID NO: 73; a heavy chain variable region of SEQ ID NO: 33 and a light chain variable region of SEQ ID NO: 74; a heavy chain variable region of SEQ ID NO: 34 and a light chain variable region of SEQ ID NO: 75; a heavy chain variable region of SEQ ID NO: 35 and a light chain variable region of SEQ ID NO: 76; a heavy chain variable region of SEQ ID NO: 36 and a light chain variable region of SEQ ID NO: 77; a heavy chain variable region of SEQ ID NO: 37 and a light chain variable region of SEQ ID NO: 78; a heavy chain variable region of SEQ ID NO: 38 and a light chain variable region of SEQ ID NO: 79; a heavy chain variable region of SEQ ID NO: 39 and a light chain variable region of SEQ ID NO: 80; a heavy chain variable region of SEQ ID NO: 40 and a light chain variable region of SEQ ID NO: 81; a heavy chain variable region of SEQ ID NO: 41 and a light chain variable region of SEQ ID NO: 82; a heavy chain variable region of SEQ ID NO: 42 and a light chain variable region of SEQ ID NO: 83; a heavy chain variable region of SEQ ID NO: 43 and a light chain variable region of SEQ ID NO: 84; a heavy chain variable region of SEQ ID NO: 44 and a light chain variable region of SEQ ID NO: 85; a heavy chain variable region of SEQ ID NO: 45 and a light chain variable region of SEQ ID NO: 86; a heavy chain variable region of SEQ ID NO: 87 and a light chain variable region of SEQ ID NO: 92; a heavy chain variable region of SEQ ID NO: 88 and a light chain variable region of SEQ ID NO: 93; a heavy chain variable region of SEQ ID NO: 89 and a light chain variable region of SEQ ID NO: 94; a heavy chain variable region of SEQ ID NO: 90 and a light chain variable region of SEQ ID NO: 95; a heavy chain variable region of SEQ ID NO: 91 and a light chain variable region of SEQ ID NO: 96; a heavy chain variable region of SEQ ID NO: 100 and a light chain variable region of SEQ ID NO: 116; a heavy chain variable region of SEQ ID NO: 101 and a light chain variable region of SEQ ID NO: 117; a heavy chain variable region of SEQ ID NO: 102 and a light chain variable region of SEQ ID NO: 118; a heavy chain variable region of SEQ ID NO: 103 and a light chain variable region of SEQ ID NO: 119; a heavy chain variable region of SEQ ID NO: 104 and a light chain variable region of SEQ ID NO: 120; a heavy chain variable region of SEQ ID NO: 105 and a light chain variable region of SEQ ID NO: 121; a heavy chain variable region of SEQ ID NO: 106 and a light chain variable region of SEQ ID NO: 122; a heavy chain variable region of SEQ ID NO: 107 and a light chain variable region of SEQ ID NO: 123; a heavy chain variable region of SEQ ID NO: 108 and a light chain variable region of SEQ ID NO: 124; a heavy chain variable region of SEQ ID NO: 109 and a light chain variable region of SEQ ID NO: 125; a heavy chain variable region of SEQ ID NO: 110 and a light chain variable region of SEQ ID NO: 126; a heavy chain variable region of SEQ ID NO: 111 and a light chain variable region of SEQ ID NO: 127; a heavy chain variable region of SEQ ID NO: 112 and a light chain variable region of SEQ ID NO: 128; a heavy chain variable region of SEQ ID NO: 113 and a light chain variable region of SEQ ID NO: 129; a heavy chain variable region of SEQ ID NO: 114 and a light chain variable region of SEQ ID NO: 130; a heavy chain variable region of SEQ ID NO: 115 and a light chain variable region of SEQ ID NO: 131, but is not limited thereto.

With respect to the objects of the present invention, the antibody or antigen binding fragment thereof may have cross-reactivity with two or more kinds of coronaviruses. The coronavirus may be SARS-CoV, SARS-CoV-2, or MERS-CoV, but is not limited thereto, as long as it has cross-reactivity.

For example, the antibody or antigen binding fragment thereof having cross-reactivity with two or more kinds of coronaviruses may include one or more heavy chain variable regions selected from the group including any one sequence of SEQ ID NOS: 6 to 12, SEQ ID NOS: 14 to 22, SEQ ID NOS: 24, 25, 32, 37, and 45, SEQ ID NOS: 87 to 91, and SEQ ID NOS: 97 to 99; and one or more light chain variable regions selected from the group including any one sequence of SEQ ID NOS: 47 to 53, SEQ ID NOS: 55 to 63, SEQ ID NOS: 65, 66, 73, 78, and 86, and SEQ ID NOS: 92 to 96, but is not limited thereto.

Specifically, it may include any one of a heavy chain variable region of SEQ ID NO: 6 and a light chain variable region of SEQ ID NO: 47; a heavy chain variable region of SEQ ID NO: 7 and a light chain variable region of SEQ ID NO: 48; a heavy chain variable region of SEQ ID NO: 8 and a light chain variable region of SEQ ID NO: 49; a heavy chain variable region of SEQ ID NO: 9 and a light chain variable region of SEQ ID NO: 50; a heavy chain variable region of SEQ ID NO: 10 and a light chain variable region of SEQ ID NO: 51; a heavy chain variable region of SEQ ID NO: 11 and a light chain variable region of SEQ ID NO: 52; a heavy chain variable region of SEQ ID NO: 12 and a light chain variable region of SEQ ID NO: 53; a heavy chain variable region of SEQ ID NO: 14 and a light chain variable region of SEQ ID NO: 55; a heavy chain variable region of SEQ ID NO: 15 and a light chain variable region of SEQ ID NO: 56; a heavy chain variable region of SEQ ID NO: 16 and a light chain variable region of SEQ ID NO: 57; a heavy chain variable region of SEQ ID NO: 17 and a light chain variable region of SEQ ID NO: 58; a heavy chain variable region of SEQ ID NO: 18 and a light chain variable region of SEQ ID NO: 59; a heavy chain variable region of SEQ ID NO: 19 and a light chain variable region of SEQ ID NO: 60; a heavy chain variable region of SEQ ID NO: 20 and a light chain variable region of SEQ ID NO: 61; a heavy chain variable region of SEQ ID NO: 21 and a light chain variable region of SEQ ID NO: 62; a heavy chain variable region of SEQ ID NO: 22 and a light chain variable region of SEQ ID NO: 63; a heavy chain variable region of SEQ ID NO: 24 and a light chain variable region of SEQ ID NO: 65; a heavy chain variable region of SEQ ID NO: 25 and a light chain variable region of SEQ ID NO: 66; a heavy chain variable region of SEQ ID NO: 32 and a light chain variable region of SEQ ID NO: 73; a heavy chain variable region of SEQ ID NO: 37 and a light chain variable region of SEQ ID NO: 78; a heavy chain variable region of SEQ ID NO: 45 and a light chain variable region of SEQ ID NO: 86; a heavy chain variable region of SEQ ID NO: 87 and a light chain variable region of SEQ ID NO: 92; a heavy chain variable region of SEQ ID NO: 88 and a light chain variable region of SEQ ID NO: 93; a heavy chain variable region of SEQ ID NO: 89 and a light chain variable region of SEQ ID NO: 94; a heavy chain variable region of SEQ ID NO: 90 and a light chain variable region of SEQ ID NO: 95; a heavy chain variable region of SEQ ID NO: 91 and a light chain variable region of SEQ ID NO: 96, but is not limited thereto.

As used herein, the term "human antibody" refers to a molecule derived from human immunoglobulin, in which amino acid sequences constituting the antibody, including complementarity-determining regions and framework regions, consist entirely of amino acid sequences of human immunoglobulin. Human antibodies are generally used in the therapy of human diseases, and have three or more potential advantages. First, human antibodies more preferably interact with the human immune system to more effectively destroy target cells by, for example, complement-dependent cytotoxicity (CDC) or antibody-dependent cell-mediated cytotoxicity (ADCC). A second advantage is that the human immune system does not recognize human antibodies as foreign molecules. A third advantage is that the half-lives of human antibodies are similar to those of naturally occurring antibodies in the human circulatory system even when they are administered in smaller doses or with less frequency. Therefore, the human antibodies according to the present invention have a prophylactic or therapeutic effect on coronavirus, particularly SARS-CoV-2, and thus may be usefully used in the treatment of SARS-CoV-2.

When the human monoclonal antibody of the present invention includes a constant region, it may include a constant region derived from IgG, IgA, IgD, IgE, or IgM, or a constant region by combinations thereof or hybrids thereof.

As used herein, the term "combination" means that a polypeptide encoding a single-chain immunoglobulin constant region of the same origin is linked to a single-chain polypeptide of a different origin, upon forming a dimer or multimer. For example, a dimer or multimer may be formed from two or more constant regions selected from the group consisting of constant regions of IgG, IgA, IgD, IgE, and IgM.

As used herein, the term "hybrid" means that sequences encoding two or more immunoglobulin heavy chain constant regions of different origins are present in a single-chain immunoglobulin heavy-chain constant region. For example, domain hybrids composed of one to four domains selected from the group consisting of CH1, CH2, CH3, and CH4 of IgG, IgA, IgD, IgE, and IgM are possible.

Further, in the present invention, the origins of variable and constant regions of the antibody or the antigen binding fragment thereof may be the same as or different from each other, and the origins of CDR, and variable and constant regions excluding the same, may be the same as or different from each other.

For example, the antibody of the present invention or the antigen binding fragment thereof may include a heavy chain variable region including a heavy chain CDR1 of SEQ ID NO: 139; a heavy chain CDR2 of SEQ ID NO: 140; and a heavy chain CDR3 of SEQ ID NO: 141, and a light chain variable region including a light chain CDR1 of SEQ ID NO: 142 or 148; a light chain CDR2 of SEQ ID NO: 143 or 146; and a light chain CDR3 of SEQ ID NO: 144 or 147, but is not limited thereto.

Specifically, it may include a heavy chain variable region including a heavy chain CDR1 of SEQ ID NO: 139; a heavy chain of CDR2 SEQ ID NO: 140; and a heavy chain CDR3 of SEQ ID NO: 141, and a light chain variable region including a light chain CDR1 of SEQ ID NO: 142; a light chain CDR2 of SEQ ID NO: 143; and a light chain CDR3 of SEQ ID NO: 144, or a heavy chain variable region including a heavy chain CDR1 of SEQ ID NO: 139; a heavy chain CDR2 of SEQ ID NO: 140; and a heavy chain CDR3 of SEQ ID NO: 141, and a light chain variable region including a light chain CDR1 of SEQ ID NO: 145; a light chain CDR2 of SEQ ID NO: 146; and a light chain CDR3 of SEQ ID NO: 147, but is not limited thereto.

Further, in the present invention, the antibody or the antigen binding fragment thereof may include a heavy chain variable region consisting of an amino acid sequence of SEQ ID NO: 103; and a light chain variable region consisting of an amino acid sequence of SEQ ID NO: 119, but is not limited thereto.

In one embodiment of the present invention, the antibody including the heavy chain variable region consisting of the amino acid sequence of SEQ ID NO: 103; and the light chain variable region consisting of the amino acid sequence of SEQ ID NO: 119 was named "M4-4" or "MG1141A".

Further, the antibody of the present invention may include a heavy chain variable region including a heavy chain FR (Framework region) 1 of SEQ ID NO: 148, 152 or 155; a heavy chain FR2 of SEQ ID NO: 149 or 156; a heavy chain FR3 of SEQ ID NO: 150, 153 or 157; and a heavy chain FR4 of SEQ ID NO: 151, 154 or 158, and a light chain variable region including a light chain FR1 of SEQ ID NO: 159, 163 or 166; a light chain FR2 of SEQ ID NO: 160 or 167; a light chain FR3 of SEQ ID NO: 161, 164 or 168; and a light chain FR4 of SEQ ID NO: 162, 165 or 169, but is not limited thereto.

Specifically, it may include a heavy chain variable region including a heavy chain FR (Framework region) 1 of SEQ ID NO: 148; a heavy chain FR2 of SEQ ID NO: 149; a heavy chain FR3 of SEQ ID NO: 150; and a heavy chain FR4 of SEQ ID NO: 151, and a light chain variable region including a light chain FR1 of SEQ ID NO: 159; a light chain FR2 of SEQ ID NO: 160; a light chain FR3 of SEQ ID NO: 161; and a light chain FR4 of SEQ ID NO: 162, or a heavy chain variable region including a heavy chain FR (Framework region) 1 of SEQ ID NO: 152; a heavy chain FR2 of SEQ ID NO: 149; a heavy chain FR3 of SEQ ID NO: 153; and a heavy chain FR4 of SEQ ID NO: 154, and a light chain variable region including a light chain FR1 of SEQ ID NO: 163; a light chain FR2 of SEQ ID NO: 160; a light chain FR3 of SEQ ID NO: 164; and a light chain FR4 of SEQ ID NO: 165, or a heavy chain variable region including a heavy chain FR (Framework region) 1 of SEQ ID NO: 155; a heavy chain FR2 of SEQ ID NO: 156; a heavy chain FR3 of SEQ ID NO: 157; and a heavy chain FR4 of SEQ ID NO: 158, and a light chain variable region including a light chain FR1 of SEQ ID NO: 166; a light chain FR2 of SEQ ID NO: 167; a light chain FR3 of SEQ ID NO: 168; and a light chain FR4 of SEQ ID NO: 169, but is not limited thereto.

Another aspect of the present invention provides a method of preparing the novel antibody or antigen binding fragment thereof.

The novel antibody of the present invention or the antigen binding fragment thereof may be easily prepared by a known technology of preparing antibodies. For example, the method may be carried out by preparing a hybridoma using B lymphocyte derived from immunized animals (Koeher and Milstein, 1976, Nature, 256:495), or by using a phage display technology, but is not limited thereto.

Antibody library using the phage display technology is a method in which antibodies are expressed on the phage surface by directly obtaining antibody genes from B lymphocyte without preparing hybridomas. When the phage display technology is used, it is possible to overcome existing difficulties associated with formation of monoclonal antibodies by B-cell immortalization. A general phage display technology includes: 1) incorporating an oligonucleotide having a random sequence into the gene site which corresponds to the N-terminal of phage coat protein pill (or pIV); 2) expressing a fusion protein of a part of natural coat protein and polypeptide encoded by the oligonucleotide with random sequence; 3) treating a receptor material capable of binding to the polypeptide encoded by the oligonucleotide; 4) eluting peptide-phage particles bound to the receptor using a molecule having a low pH or bonding competitiveness; 5) amplifying the eluted phage in host cells by panning; 6) repeating the above process to obtain a desired level; and 7) identifying a sequence of active peptide from DNA sequences of phage clones selected by panning.

Still another aspect of the present invention provides a polynucleotide encoding the novel antibody, an expression vector including the polynucleotide, and a transformant including the expression vector.

Still another aspect of the present invention provides an expression vector including the antibody provided in the present invention or the antigen binding fragment thereof, and a host cell introduced with the vector.

The antibody is the same as described above.

The expression vector including the polynucleotide encoding the antibody provided in the present invention may include, but is not particularly limited to, a vector that allows replication and/or expression of the polynucleotide in eukaryotic or prokaryotic cells such as mammalian cells (*e.g*., human, monkey, rabbit, rat, hamster, mouse cells, *etc*.), plant cells, yeast cells, insect cells, or bacterial cells (*e.g*., *E. coli*, *etc*.). Specifically, the vector may be a vector that is operably linked to a suitable promoter so as to induce the expression of the polynucleotide in the host cell, and includes at least one selection marker. For example, the polynucleotide may be introduced into a phage, a plasmid, a cosmid, a mini-chromosome, a virus, or a retroviral vector.

The expression vector including the polynucleotide encoding the antibody may be an expression vector including respective polynucleotides encoding the heavy chain or the light chain of the antibody, respectively, or an expression vector including both the polynucleotides encoding the heavy chain and the light chain.

The transformant introduced with the expression vector provided in the present invention may include, but is not particularly limited to, bacterial cells such as *E. coli*, *Streptomyces*, *Salmonella typhimurium*, *etc*.; yeast cells; fungus cells such as *Pichia pastoria*, *etc*.; insect cells such as drosophila, spodoptera Sf9, *etc*.; animal cells such as CHO (Chinese hamster ovary cells), SP2/0 (mouse myeloma), human lymphoblastoid, COS, NSO (mouse myeloma), 293T, Bowes melanoma cells, HT-1080, BHK (baby hamster kidney cells), HEK (human embryonic kidney cells), and PERC.6 (human retina cell), *etc*.; and plant cells, which are transformed by introduction of the expression vector.

As used herein, the term "introduction" means the delivery of the vector including the polynucleotide encoding the antibody into host cells. The introduction may be carried out using various methods well known in the art, including calcium phosphate-DNA co-precipitation, DEAE-dextran-mediated transfection, polybrene-mediated transfection, electroporation, microinjection, liposome fusion, lipofectamine transfection, protoplast fusion, *etc.* Further, transduction refers to a process whereby a target material is transferred to cells via viral particles by means of infection. In addition, the vector may be introduced into host cells by gene bombardment. In the present invention, introduction may be used interchangeably with transformation.

Still another aspect of the present invention provides a composition for diagnosing coronavirus infection, the composition including the antibody provided in the present invention or the antigen binding fragment thereof.

Still another aspect of the present invention provides a kit for diagnosing coronavirus infection, the kit including the antibody provided in the present invention or the antigen binding fragment thereof.

Still another aspect of the present invention provides a method of diagnosing coronavirus infection or a method of providing information for diagnosing coronavirus infection, the method including the step of detecting coronavirus present in a biological sample separated from an individual suspected of having coronavirus infection using the antibody provided in the present invention or the antigen binding fragment thereof; or the composition for diagnosing the infection, the composition including the antibody or the antigen binding fragment thereof; or the kit including the composition.

As used herein, the term "diagnosing" includes determining susceptibility of an individual for a specific disease or disorder, determining whether or not an individual has a specific disease or disorder, or monitoring an individual's conditions to provide information about therapeutic efficacy.

With respect to the objects of the present invention, the diagnosing may be to determine whether or not the coronavirus infectious disease has occurred. The "coronavirus infectious disease" refers to a disease caused by an infection in which the coronavirus invades the body of a host organism. Specifically, the coronavirus may be SARS-CoV-2 (SARS-coronavirus-2). For example, the coronavirus infectious disease may be COVID-19.

The composition for diagnosing coronavirus infection of the present invention may include other antibody or antigen binding fragment thereof than the antibody of the present invention or the antigen binding fragment thereof. In addition, the composition may further include a substance needed for an antigen binding reaction, for example, a reagent, together with the antibody of the present invention or the antigen binding fragment thereof. In addition, the composition may further include a substance needed for detecting this binding, a substance for stabilizing the antibody of the present invention or the antigen binding fragment thereof, *etc.*

For example, the antibody or the antigen binding fragment thereof included in the composition for diagnosing of the present invention may be labeled for detection. Various methods that may be used to label molecules are well known to those skilled in the art, and are considered to fall within the scope of the present invention.

Examples of labels that may be used in the present invention may include enzymes, radioisotopes, colloidal metals, fluorescent compounds, chemiluminescent compounds and bioluminescent compounds. Commonly used labels include fluorescent substances (*e.g*., fluorescein, rhodamine, Texas red, *etc*.), enzymes (*e.g*., horseradish peroxidase, β-galactosidase, or alkaline phosphatase), radioisotopes (*e.g.,* ³²P or ¹²⁵I), biotin, digoxigenin, colloidal metals, or chemiluminescent or bioluminescent compounds (*e.g*., dioxetane, luminol or acridinium). Labeling methods, such as covalent bonding, iodination, phosphorylation, or biotinylation of enzymes or biotinyl groups, are also well known in the art.

The kit of the present invention may further include reagents, tools, *etc.* needed for detecting the binding of an antigen with the antibody of the present invention or the antigen binding fragment thereof.

For example, the kit container of the present invention may include a solid carrier. The antibody of the present invention may be attached to the solid carrier, and such a solid carrier may be porous, non-porous, planar, or non-planar.

For example, the kit may include the solid carrier; a sample pad receiving a sample to be analyzed and having a buffer input unit and a sample input unit; a conjugate pad containing an antibody that specifically binds to coronavirus contained in the sample introduced from the sample pad; a signal detection pad including a signal detection unit for detecting whether or not the coronavirus is present in the sample and a control unit for identifying whether or not the sample has moved to an absorbent pad regardless of the presence or absence of the analyte; and the absorbent pad absorbing the sample in which the signal detection reaction has been completed, but is not limited thereto.

For example, the kit of the present invention may be in the form of an ELISA (enzyme-linked immunosorbent assay) kit. As used herein, the term "ELISA" is also called enzyme-linked immunosorbent assay, and refers to a quantification method of using absorbance through a reaction between an enzyme and a substrate after forming an antigen-antibody complex by binding the enzyme to the antibody. ELISA includes a direct ELISA of using a labeled antibody which recognizes an antigen adhered to a solid support body, an indirect ELISA of using a labeled secondary antibody which recognizes a captured antibody of an antigen-antibody complex wherein the antigen adhered to a solid support body, a direct sandwich ELISA of using another labeled antibody which recognizes an antigen of an antigen-antibody complex adhered to a solid support body, and an indirect sandwich ELISA of using a labeled secondary antibody which recognizes an antibody, after reacting with the antibody which recognizes an antigen of an antigen-antibody complex adhered to a solid support body, *etc.*

The antibody of the present invention or the fragment thereof may be used in detecting the presence of coronavirus in a biological sample by bringing the antibody of the present invention or the fragment thereof into contact with the biological sample and then by examining the reaction therebetween.

The biological sample may be any one selected from the group consisting of sputum, saliva, blood, sweat, lung cells, mucus of lung tissue, respiratory tissue and saliva of a subject, but is not limited thereto, and the biological sample may be prepared using a common method known to those skilled in the art.

For example, the method of providing information for diagnosing coronavirus infection of the present invention may include the steps of (a) bringing the antibody or the antigen binding fragment thereof provided in the present invention into contact with a biological sample; and (b) detecting a complex of the antibody or the antigen binding fragment thereof and a coronavirus spike protein, which is formed by the contact. Specifically, in the step (b), the complex of the antibody or the fragment thereof and the coronavirus spike protein may be a complex with a connector domain (CD) of the SARS-CoV-2 spike protein.

As used herein, the term "antigen-antibody complex" refers to a combination material of the corresponding protein antigen in the sample and the antibody recognizing the antigen. Detection of the antigen-antibody complex may be performed by a method known in the art, for example, spectroscopic, photochemical, biochemical, immunochemical, electrical, optical, chemical, and other methods, and specifically, by any method selected from the group consisting of a colorimetric method, an electrochemical method, a fluorimetric method, luminometry, a particle counting method, visual assessment, and a scintillation counting method. A method such as Western blotting, ELISA, radioimmunoassay, radioimmunodiffusion, Ouchterlony radial immunodiffusion, rocket immunoelectrophoresis, immunohistochemical staining, an immunoprecipitation assay, a complete fixation assay, fluorescence-activated cell sorting (FACS), a protein chip, *etc.* may be used, but is not limited thereto.

Still another aspect of the present invention provides a pharmaceutical composition for preventing or treating coronavirus infection, the pharmaceutical composition including the antibody or the antigen binding fragment thereof. Specifically, the coronavirus may be SARS-CoV-2. For example, the composition may be for the prevention or treatment of COVID-19.

As used herein, the term "COVID-19" refers to a disease caused by SARS-CoV-2 infection. Since the antibody of the present invention exhibits neutralization and infection inhibition ability against SARS-CoV-2, it may be used for the prevention or treatment of COVID-19.

As used herein, the term "prevention" may refer to any action resulting in the suppression or delay of the onset of coronavirus infection by the administration of the composition, and the term "treatment" may refer to any action resulting in the improvement or beneficial alteration of the symptoms caused by coronavirus infection by the administration of the composition. The pharmaceutical composition may further include a pharmaceutically acceptable carrier.

As used herein, the term "pharmaceutically acceptable carrier" refers to a carrier or diluent that does not cause significant irritation to an organism and does not abrogate the biological activity and properties of the administered compound. For liquid formulation, the pharmaceutically acceptable carriers in the formulated composition should be sterilized and suitable to living bodies. A saline solution, sterilized water, Ringer's solution, a buffered saline solution, an albumin injection solution, a dextrose solution, a maltodextrin solution, glycerol, ethanol, or a mixture of one or more thereof may be used. As needed, other common additives may be added, such as antioxidants, buffers, bacteriostatic agents or the like. Also, diluting agents, dispersing agents, surfactants, binders or lubricants may be further added to formulate the composition to injectable formulations such as an aqueous solution, a suspension, and an emulsion, *etc*., pills, capsules, granules, or tablets.

The pharmaceutical composition may be in various oral or non-oral dosage forms. The pharmaceutical composition may be formulated using a diluent or excipient commonly used, such as a filler, a thickener, a binder, a wetting agent, a disintegrant, a surfactant, *etc.* Solid preparations for oral administration may include tablets, pills, powders, granules, capsules, *etc.* These solid preparations may be prepared by mixing one or more compounds with one or more excipients, for example, starch, calcium carbonate, sucrose, lactose, or gelatin, *etc.* In addition to a simple excipient, a lubricant such as magnesium stearate, talc, *etc.* may be used. Liquid preparations for oral administration may include suspensions, solutions for internal use, emulsion, syrups, *etc.* In addition to a simple diluent such as water or liquid paraffin commonly used, various excipients, for example, wetting agents, sweeteners, aromatics, preservatives, *etc.* may be included. Also, preparations for parenteral administration include sterile aqueous solutions, non-aqueous solvents, suspensions, emulsions, lyophilized preparations, and suppositories. Propylene glycol, polyethylene glycol, vegetable oils such as olive oil, and injectable esters such as ethyl oleate may be used for the non-aqueous solvents and suspensions. As base materials of suppositories, Witepsol, macrogol, Tween 61, cacao butter, laurin butter, glycerogelatin, *etc.* may be used.

The pharmaceutical composition may have any one formulation selected from the group consisting of tablets, pills, powders, granules, capsules, suspensions, solutions for internal use, emulsion, syrups, sterile aqueous solutions, non-aqueous solvents, suspensions, emulsions, lyophilized preparations, and suppositories.

The composition of the present invention is administered in a pharmaceutically effective amount.

As used herein, the term "pharmaceutically effective amount" refers to an amount sufficient for treating disease in a reasonable ratio of advantage/risk, which is applicable to medical treatment. The level of the effective dosage may be determined according to a subject's kind and severity, age, gender, the type of cancer, drug activity, sensitivity to the drug, the time of administration, the route of administration, the rate of excretion, treatment duration, or elements including drugs that are concurrently administered, and other elements well-known in the medical field. The composition of the present invention may be administered singly or in combination with other therapeutic agents, or may be also administered with existing therapeutic agents in a sequential or simultaneous manner. Also, the composition may be administered in a single dose or may be divided into multiple doses. It is important to administer the composition in the minimum amount that may achieve the maximum effect without causing side effects, in view of all of the above-described factors, and it may be easily determined by those skilled in the art.

Still another aspect of the present invention provides a method of preventing or treating coronavirus infection using the antibody.

Specifically, the coronavirus may be SARS-CoV-2.

For example, the method may include the step of administering the antibody to an individual suspected of having SARS-CoV-2 infection.

The method may be a method including the step of administering the pharmaceutical composition including the antibody and the pharmaceutically acceptable carrier to an individual which is infected with coronavirus or has been infected with coronavirus. The pharmaceutically acceptable carrier is as described above.

The individual may be a mammal, such as a cow, pig, sheep, chicken, dog, human, *etc*., and a bird, and it may include any individual, of which coronavirus infection may be treated by administration of the composition of the present invention, without limitation. In this regard, the antibody may be administered in single or multiple doses in a pharmaceutically effective amount. In this regard, the antibody may be administered in the form of a solution, powder, aerosol, capsule, enteric-coated tablet or capsule, or suppository. The route of administration includes intraperitoneal administration, intravenous administration, intramuscular administration, subcutaneous administration, intradermal administration, oral administration, topical administration, intranasal administration, intrapulmonary administration, and intrarectal administration, but is not limited thereto.

The method may also be used for the prevention or treatment of coronavirus infectious diseases, specifically, SARS-CoV-2 infectious diseases. For example, the method may be used for the prevention or treatment of COVID-19, but is not limited thereto.

Still another aspect of the present invention provides use of the antibody in the preparation of pharmaceuticals for preventing or treating coronavirus infection. The pharmaceuticals may be used for the prevention or treatment of SARS-CoV-2 infectious diseases, for example, COVID-19, but are not limited thereto. The antibody and the prevention or treatment of SARS-CoV-2 infection are as described above.

Hereinafter, the present invention will be described in more detail with reference to the following Examples. However, the following Examples are only for illustrative purposes, and the scope of the present invention is not intended to be limited by these Examples.

### Experimental Example 1. Expression and Purification of Recombinant Protein

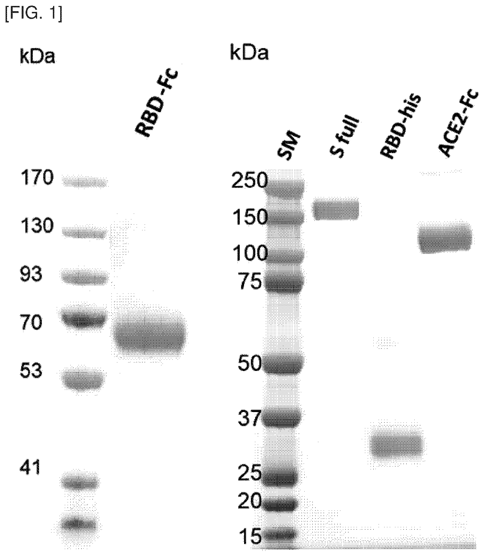

Genes encoding the ecto-domain (spike-ECD) and the RBD (RBD-his) of SARS-CoV-2 spike glycoprotein were synthesized and cloned into a pCIW mammalian expression vector with a C-terminal 6× histidine tag. Genes encoding RBD-Fc and human ACE2-Fc were cloned into the pCIW vector. The pCIW mammalian vectors were transfected into Expi293F^{™} cells (Cat. No. A1435101). All the steps for expression analysis were performed according to the manufacturer's instructions. After 5 days to 6 days of cell culture, the cells were harvested by centrifugation, and the supernatant was passed through a 0.22 µm filter to remove cell debris. For Histag purification of recombinant proteins, a Ni-NTA resin was added to the supernatant. MabSelect Xtra (Cat. No. 17-5269-02, GE Healthcare) was used as a resin for protein-A purification of Fc-fused proteins. All purification procedures were performed according to the manufacturer's instructions. The buffer of eluted proteins was changed with phosphate-buffered saline (PBS) using Zeba Spin Desalting Columns. Protein concentration was quantified using a Nanodrop 2000C spectrophotometer (Thermo Scientific) (FIG. 1).

### Experimental Example 2. Production of Antibodies Against SARS-CoV-2 Spike Protein

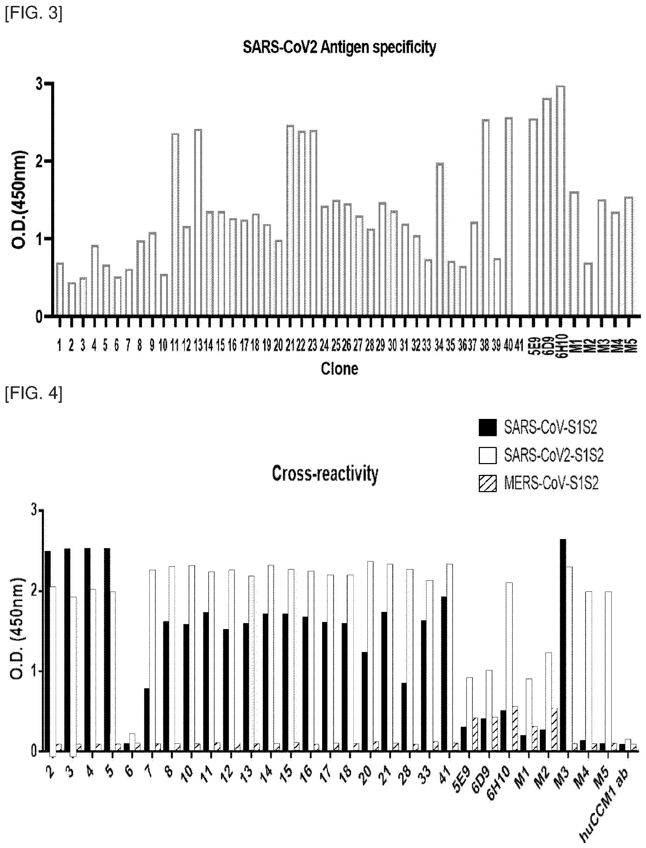

General antibody production techniques were used to produce antibodies against SARS-CoV-2 spike protein. Specifically, a human B cell-derived library, a mouse immune library, and a synthetic human domain antibody library were constructed and screened. For screening, a commonly used single clone ELISA method was used. The single clone ELISA screening method is a method of mixing the spike protein with an antibody expressed in a single clone to examine whether or not the corresponding antibody is specific to the spike protein. As a result of the screening, spike protein-specific clones of 41 (clones 1 to 41), 5 (clones M1 to M5), and 3 (clone 5E9, 6D9, 6H10) were obtained from the human B cell-derived library, the mouse immune library, and the synthetic human domain antibody library, respectively. Through the sequence analysis, sequences of heavy and light chains of the obtained antibodies were identified (FIG. 3, Tables 1, 2, and 3). However, with regard to the clones obtained from the synthetic human domain antibody library, only the heavy chain sequence information was included, because the synthetic library itself is a library consisting of only heavy chain sequences.

**[Table 1]**

| Clone | Variable region | Amino acid sequence | SEQ ID NO. |
|---|---|---|---|
| 1 | Heavy chain | EVQLVQSGAEVQKPGSSVKVSCKASGGTFSSYPISWVRQAPGQG LEWMGKIIPILGIPNYAQRFQGRVTITADKSTGTAYLDLSSLSSEDT AVYYCARAGGYSGYGAHYYMDVWGKGTLVTVSS | 5 |
| | Light chain | QSVLTQPPSVSGAPGQRVTISCTGSSSNIGAGYDVHWYQQLPGT APKLLIYGNSNRPSGVPDRFSGSKSGTSASLAITGLQAEDEADYY CQSYDSSLSGSVFGGGTKLTVLG | 46 |
| 2 | Heavy chain | EVQLVESGGGLIQPGGSLRLSCAASGFTFSNFAMSWVRQAPGKG LEWVSGISGSGGSTSYADSVKGRFTISRDNSKNTLYLQMNSLRAE DTAVFYCAKDLYGGPGSSTFDYWGQGTLVTVSS | 6 |
| | Light chain | DIVMTQSPSSLSASVGDRVTITCQASQDISNYLNWYQQKPGKAPK LLIYDASNLETGVPSRFSGSGSGTDFTFTISSLQPEDIATYYCQQY DNLPLTFGPGTKVDIKR | 47 |
| 3 | Heavy chain | QVQLVQSGGGLIQPGGSLRLSCAASGFTFSNFAMSWVRQAPGK GLEWVSGISGSGGSTSYADSVKGRFTISRDNSKNTLYLQMNSLRA EDTAVFYCAKDLYGGPGSSTFDYWGQGTLVTVSS | 7 |
| | Light chain | AIWMTQSPSSLSASVGDRVTITCQASQDISNYLNWYQQKPGKAPK LLIYDASNLETGVPSRFSGNGSGTDFTLTISSLQPEDFAIYYCQQS YSTPLTFGGGTKVEIKR | 48 |
| 4 | Heavy chain | EDQLVESGGGLIQPGGSLRLSCAASGFTFSNFAMSWVRQAPGKG LEWVSGISGSGGSTSYADSVKGRFTISRDNSKNTLYLQMNSLRVE DTAVFYCAKDLYGGPGSSTFDYWGQGTLVTVSS | 8 |
| | Light chain | DIVMTQTPSSLSASVGDRVTITCQASQDISIYLNWYQQKPGKAPKL LIYDASILETGVPSRFSGSGSGTDFTLTISSLQPEDFATYYCQQYFS YPITFGGGTKVEIKR | 49 |
| 5 | Heavy chain | EDQLVESGGGLIQPGGSLRLSCAASGFTFSNFAMSWVRQAPGKG LEWVSGISGSGGSTSYADSVKGRFTISRDNSKNTLYLQMNSLRAE DTAVFYCAKDLYGGPGSSTFDYWGQGTLVTVSS | 9 |
| | Light chain | DIQMTQSPSSLSASVGDRVTITCQASEDITDYLNWYQQKPGKAPK LLIYDASILETGVPSRFSGSGSGTDFTLTISSLQPEDVATYYCQQYD GLPLFGPGTKVDIKR | 50 |
| 6 | Heavy chain | QLQLQESGGGWVQPGRSLRLTCATSGFTFDDYAMHWVRQAPGK GLEWVSGITWNSDSIGY ADSVKG RFTISRDDAKNSL YLQMDNLRP EDTALYYCVKDSAYRILPYWYFDLWGRGTTVTVSS | 10 |
| | Light chain | DIQMTQSPSSLSASVGDRVTITCRASQSISNYLNWYQQIPGKAPKL LMYAATRLHSGVPSRFSGSGSGTDFTLTIYSLQPEDFATYYCQQS FGSPYTFGQGTKLEIKR | 51 |
| 7 | Heavy chain | EVQLVESGGDLVQPGGSLRISCAASGFAVSNNYMSWVRQAPGK GLEWVSVIHTDGSTYYADSVKGRFTISRDNSKNTLYLQMNSLRAE | 11 |
| | | DTAVYYCAREGFGELLGSDAFDIWGQGTMVTVSS | |
| | Light chain | AIQMTQSPSSLSASVGDRVTITCRASQSISSYLNWYQQKPGKAPK LLIYAASSLQSGVPSRFSGSGSGTDFTLTISSLQPEDFATYYCQQS YSTPHTFGPGTKVDIKR | 52 |
| 8 | Heavy chain | QVQLVQSGGGLVQPGGSLRLSCVASGFTFRTFEMNWVRQAPGK GLEWVSYISPSGSSIYNADSVKGRFTISRDNARNSLYLQMNSLRA EDTAVYYCAREGGSYYGGSAFDIWGQGTMVTVSS | 12 |
| | Light chain | SYELTQPSSASGTPGQRVTISCSGSSSNIGSNTVNWYQQLPGTAP KLLIYSNNQRPSGVPDRFSGSKSGTSASLAISGLQSEDEADYYCA AWDDSLNGWVFGGGTKLTVLG | 53 |
| 9 | Heavy chain | EVQLVESGGGVVQPGRSLRLSCVASGFTFRTFEMNWVRQAPGK GLEWVSYISPSGSSIYNADSVKGRFTISRDNARNSLYLQMNSLRA EDTAVYYCAREGGSYYGGSAFDIWGQGTTVTVSS | 13 |
| | Light chain | SYELTQPPSASGTPGQRVTISCSGSSSNIERNIVNWYQQVPGTAP KLLIYTNSHRPSGVPDRFSGSKSGSAASLAISGLQSEDEADYYCAA WDDTLNGWVFGGGTKLTVLG | 54 |
| 10 | Heavy chain | EVQLVQSGGGSVQPGGSLRLSCAASGFTFRTFEMNWVRQAPGK GLEWVSYISPSGSSIYNADSVKGRFTISRDNARNSLYLQMNSLRA EDTAVYYCAREGGSYYGGSAFDIWGQGTMVTVSS | 14 |
| | Light chain | QSVPTQPPSASGTPGQRVTISCSGSSSNIGSNTVNWYQQLPGTA PKLLIYSNNQRPSGVPDRFSGSKSGTSASLAISGLQSEDEADYYC AAWDDSLNGRWVFGGGTKLTVLG | 55 |
| 11 | Heavy chain | EVQLVESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDHWGQGTLVTVSS | 15 |
| | Light chain | QSALTQPRSVSGSPGQSVTISCTGTSSDVGGYNYVSWYQQHPG KAPKLMIYDVSKRPSGVPDRFSGSKSGNTASLTISGLQAEDEADY YCCSYAGSYTWVFGGGTKLTVLG | 56 |
| 12 | Heavy chain | EVQLVESGGGLVQPGGSLRLSCAASGFTFSTYAMSWVRQAPGK GLEWVSAISGNGGSTSYAASVQGRFTISRDNSKNTLYLQMNSLRA EDTAVYYCAREGGYYYGSGSYYKPKVVFDYWGQGTLVTVSS | 16 |
| | Light chain | QSVLTQPPSVSGAPGQRVTISCTGSSSNIGAGYDVHWYQQLPGT APKLLIYGNSNRPSGVPDRFSGSKSGTSASLAITGLQAEDEADYY CQSYDSSLSGYVFGTGTKVTVLG | 57 |
| 13 | Heavy chain | QVQLVESGGGVVQPGRSLRLSCAASGFTFRGYAMHWVRQAPGK GLEWVAVISEDGSDEYYADSVKGRFTISRDNSKNTLYLQMNSLRA EDTAVYYCAREGGYYYGSGSYYKPKVVFDYWGQGTLVTVSS | 17 |
| | Light chain | QSVLTQPPSVSGAPGQRVTISCTGSSSNIGAGYDVHWYQQLPGT APKLLIYGNSNRPSGVPDRFSGSKSGTSASLAITGLQAEDEADYY | 58 |
| | | CQSYDSSLSGYVFGTGTELTVLG | |
| 14 | Heavy chain | EVQLVESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 18 |
| | Light chain | SYELTQPPSVSGAPGQRVTISCTGSSSNIGAGYDVHWYQQLPGT APKLLIYGNSNRPSGVPDRFSGSKSGTSASLAITGLQAEDEADYY CQSYDSSLSGYVFGTGTKVTVLG | 59 |
| 15 | Heavy chain | QVQLVESGGTLVQPGGSLRLSCAASGFTFSGYAMNWVRQAPGK GLEWVSSVSGRGDTTHYADSVKGRFTISRDNRENKLYLQMHSLR AEDTAVYYCAREGGYYYGSGSYYKPKVVFDYWGQGTLVTVSS | 19 |
| | Light chain | QAGLTQPPSVSGAPGQRVTMSCTGTTSNIGAGYDVHWYQQLPG TAPKLLIYGNSNRPSGVPDRFSGSKSGTSASLAISGLQSEDEADYY CAAWDDSLNGYVFGTGTKVTVLG | 60 |
| 16 | Heavy chain | EVQLVESGGGLVQPGGSLRLSCAASGFTFRTYAMSWVRQAPGK GLEWVSAISSSDDTTYYADSVKGRFTISRDNSKNTLYLQMNSLRA EDTAVYYCAREGGYYYGSGSYYKPKVVFDYWGQGTLVTVSS | 20 |
| | Light chain | SYELTQPLSVSGAPGQRVTISCTGSSSNIGAGYDVHWYQQLPGTA PKLLIYGNSNRPSGVPDRFSGSKSGTSASLAITGLQAEDEADYYC QSYDSSLSGWVFGGGTKLTVLG | 61 |
| 17 | Heavy chain | EVQLVESGGGLVQPGGSLRLSCAASGFSSSYYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 21 |
| | Light chain | QSVLTQPPSVSGAPGQRVTISCTGSSSNIGAGYDVHWYQQLPGT APKLLIYGNSNRPSGVPDRFSGSKSGTSASLAITGLQAEDEADYY CQSYDSSLSGWVFGGGTKLTVLG | 62 |
| 18 | Heavy chain | EVQLVQSGGGLVQPGGSLRLSCAASGFTFRTYAMSWVRQAPGK GLEWVSAISSSDDTTYYADSVKGRFTISRDNSKNTLYLQMNSLRA EDTAVYYCAREGGYYYGSGSYYKPKVVFDYWGQGTLVTVSS | 22 |
| | Light chain | QSVLTQPPSVSGAPGQRVTISCTGSSSNIGAGYDVHWYQQLPGT APKLLIYGNSNRPSGVPDRFSGSKSGTSASLAITGLQDEDEADYY CQSYDSSLSEYVFGTGTKVTVLG | 63 |
| 19 | Heavy chain | EVQLLESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 23 |
| | Light chain | QSVLTQPPSVSGAPGQRVTISCTGSSSNFGAGYDVHWYQQLPGT APKLLIYGNDNRPSGVPDRFSGSKSGTSASLAITGLQDEDEADYY CQSYDSSLSEYVFGTGTQLTDLG | 64 |
| 20 | Heavy chain | EVQLVESGGGLVQPGGSLRLSCAASGFTFRTYAMSWVRQAPGK GLEWVSAISSSDDTTYYADSVKGRFTISRDNAKNSLYLQMNSLRA | 24 |
| | | EDTAVYYCAREGGYYYGSGSYYKPKVVFDYWGQGTTVTVSS | |
| | Light chain | QSALIQPRSVSGSPGQSVTISCTGTSSNVGVYNYVSWYQQHPGK APKLIIYDVTKRPSGVPDRFSGSKSGNTGSLTISGLQAEDEADYYS CSYAGSYTWVFGPGTKVTVLS | 65 |
| 21 | Heavy chain | QVQLLESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 25 |
| | Light chain | QTVVTQPPSVSGAPGQRVTISCTGSSSNIGAGYDVHWYQQLPGT APKLLIYGNSNRPSGVPDRFSGSKSGTSASLAITGLQAEDEADYY CQSYDSSLSGSGVFGGGTKLTVLG | 66 |
| 22 | Heavy chain | EVQLVESGGGLAQSGGSLRLSCEASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 26 |
| | Light chain | SYELTQPPSASGTPGQRVTISCSGSSSNIGAGYDVHWYQQLPGT APKLLIYGNSNRPSGVPDRFSGSKSGTSASLAITGLQAEDEADYY CQSYDYNLSGYVSGTGTKVTVLG | 67 |
| 23 | Heavy chain | QVQLVESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 27 |
| | Light chain | TYELSQPPSVSGAPGQRVTISCTGSSSNIGADYDVHWYQQLPGT APKLLIYGNNNRPSGVPDRFSGSKSGTSGSLAITGLRSEDEADYY CAAWDDSLSGYVFGTGTKVTVLG | 68 |
| 24 | Heavy chain | EVQLVESGGAMVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 28 |
| | Light chain | QPVLTQPPSVSGAPGQRVTITCTGSGSNIGAGYDVHWYQQLPGT APKLLIYGNSNRPSGAPDRVSGSKSGTSASLAITGLQAEDEADYY CQSYDSSLRTYVFGTGTKVTVLG | 69 |
| 25 | Heavy chain | EVQLVESGGGLVQPGGSLRLSCAASGFTFSTYWMSWVRQAPGK GLEWVANIKQEGSEKYYVDSVKGRFTISKDNAKNSLYLQMNSLRA EDTAVYYCAREGGYYYGSGSYYKPKVVFDYWGQGTLVTVSS | 29 |
| | Light chain | QSVLTQPPSVSGAPGQRVTISCTGSSSNIGAGYDVHWYQQLPGT APKLLIYGNSNRPSGVPDRFSGSKSGTSASLAITGLQAEDEADYY CQSYDSSLSGRVFGGGTKLTVLG | 70 |
| 26 | Heavy chain | EVQLVESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 30 |
| | Light chain | QSVLTQPPSVSGAPGQRVTISCTGSSSNIGAGYDVHWYQQLPGT APKLLIYGNSNRPSGVPDRFSGSKSGTSASLAITGLQAEDEADYY | 71 |
| | | CQSYDSNLSGYVFGTGTKVTVLG | |
| 27 | Heavy chain | EVQLVESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 31 |
| | Light chain | SYELMQPPSVSGAPGQRVTISCTGSSSNIGAGYDVHWYQQLPGT APKLLIYGNSNRPSGVPDRFSGSKSGTSASLAITGLQAEDEADYY CQSYDSSLRAYVFGTGTKVTDLG | 72 |
| 28 | Heavy chain | QVQLVQSGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 32 |
| | Light chain | DVVMTQSPSSLSASVGDRVTITCRASQGITNDLAWYQQKPGKAP KLLIYAASTLQSGVPSRFSGSGSGTDFTLTISSLQPEDFATYYCQQ SYDTPPYTFGQGTKLEIKR | 73 |
| 29 | Heavy chain | QVQLVESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 33 |
| | Light chain | QSVLTQPPSVSGAPGQRVTISCTGSSSNIGAGYDVHWYQQLPGT APKLLIYGNSNRPSGVPDRFSGSKSGTSASLAITGLQAEDEADYY CQSYDSSLSGSTVFGGGTELTVLG | 74 |
| 30 | Heavy chain | EVQLVETGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 34 |
| | Light chain | SYELTQPPSVSGAPGQRVTISCTGSSSNIGAGYDVHWYQQLPGT APKLLIYGNSNRPSGVPDRFSGSKSGTSASLAITGLQAEDEADYY CQSYDNSLSGVFGGGTKLTVLG | 75 |
| 31 | Heavy chain | EVQLVESGGGLVQPGGSLRLSCAASGFTFSTYWMSWVRQAPGK GLEWVANIKQEGSEKYYVDSVKGRFTISRDNAKNSLYLQMNSLRA EDTAVYYCAREGGYYYGSGSYYKPKVVFDYWGQGTLVTVSS | 35 |
| | Light chain | QSVLTQPPSVSGAPGQRVTISCTGSSSNIGAGYDVHWYQQVPGT APKLLIYGNINRPSGVPDRFSGSKSGTSASLAITGLQAEDEADYYC QSYDSSLSGSYVFGTGTKVTVLG | 76 |
| 32 | Heavy chain | QVQLVQSGGGVVQPGGSLRLSCAASGFTFSGYGMNWVRQAPG KGLEWVADIEKDGGERNYADSVKGRFTISRDNAKNSLYLQMNSLR AEDTAVYYCAREGGYYYGSGSYYKPKVVFDYWGQGTLVTVSS | 36 |
| | Light chain | QSVLTQPPSVSGAPGQRVTISCTGSSSNIGAGYDVHWYQHLPGT APKLLIYGNSNRPSGVPDRFSGSKSGTSASLAITGLQAEDEADYY CQSYDGSLSGWVFGGGTKLTVLG | 77 |
| 33 | Heavy chain | EVQLVESGGGLIQPGGSLRLSCAASGFTFSSFWMSWVRQAPGK GLEWVANIKQDGSEKYSVDSVKGRFTISRDNAKNSLYLQMNSLRA | 37 |
| | | EDTAVYYCARHGIYCSGDNCYYFAPTLSSHAFDIWGQGTMVTVSS | |
| | Light chain | AIQLTQSPSSLSASVGDRVTITCRASQSISSYLNWYQQKPGKAPKL LIYAASSLQSGVPSRFSGSGSGTDFTLTISSLQPEDFATYYCQQSY STPLTFGGGTKVEIKR | 78 |
| 34 | Heavy chain | QVQLVQSGGGLVQPGRSLRLSCAASGFTFDDYAMHWVRQAPGK GLEWVSGSSWNSGTIGYADSVKGRFTISRDNAKNSLYLQMNSLR AEDTALYYCAIIPGDGYNYPGASDFDYWGQGTLVTVSS | 38 |
| | Light chain | QPVLTQPPSASGTPGQRVIISCSGSSSNIGSHTVNWYQQLPGTAP KLLIYNNNQRPSGVPDRFSGSKSGTSASLAISGLQSADEADYSCA AWDDSLNGYVFGTGTKVTVLG | 79 |
| 35 | Heavy chain | EVQLLESGGGLVQPGRSLKLSCAASGFTFDDYAMHWVRQAPGK GLEWVSGSSWNSGTIGYADSVKGRFTISRDNAKNSLYLQMNSLR AEDTALYYCAIIPGDGYNYPGASDFDYWGQGTLVTVSS | 39 |
| | Light chain | QSALTQPASVSGSPGQSITISCTGTSSDVGGYNYVSWYQQHPGK APKLMIYDVGQRPSGVSNRFSGSKSGNTASLTISGLQAEDEADYY CSSYTSSSSYVFGTGTKVTVLG | 80 |
| 36 | Heavy chain | QVTLKESGGGLVQPGRSLRLSCAASGFTFDDYAMHWVRQAPGK GLEWVSGSSWNSGTIGYADSVKGRFTISRDNAKNSLYLQMNSLR AEDTALYYCAIIPGDGYNYPGASDFDYWGQGTLVTVSS | 40 |
| | Light chain | SYELTQPPSVSVAPGKTARITCGGNNIGSKSVHWYQQKPGQAPV LVVYDDSDRPSGIPERFSGSNSGNTATLTISRVEAGDEADYYCQV WDSSSDHVVFGGGTQLIILG | 81 |
| 37 | Heavy chain | QVQLVESGGGLVQPGGSLRLSCAASGFTFDDYAMHWVRQAPGK GLEWVSGSSWNSGTIGYADSVKGRFTISRDNAKNSLYLQMNSLR AEDTALYYCAIIPGDGYNYPGASDFDYWGQGTTVTVSS | 41 |
| | Light chain | RPVLTQPPSASGTPGQRATISCSGSSSNIGSNTVNWYRQLPGTAP KLLIHSNNQRPSGVPDRFSGSKSGTSASLAISGLQSEDEADYYCA TWDDSLDGWVFGGGTKLTVLG | 82 |
| 38 | Heavy chain | QVQLVQSGAEVKKPGASVRVSCKASGYTFSSYYTHWVRQAPGQ GLEWMGIINPSGGSTIYAQKFQGRVTMTRDTSTSTVYMELSSLRS EDTAVYYCARLPRDGVHASDIWGQGTTVTVSS | 42 |
| | Light chain | QTVVTQEPSVSAAPEQKVTISCSGSSSNIGNNYVSWYQQLPGTAP KLLIYDNNKRPSGIPDRFSGSKSGTSATLGITGLQTGDEADYYCGT WDSSLSAYVFGTGTKVTVLG | 83 |
| 39 | Heavy chain | EVQLVESGGGLVQPGGSLRLSCAASGFTFSTYYMGWVRQAPGK GLEWVSLISGSGGSTYYDDSVKGRFTISRDNSKNTLYLQMNSLRA EDTAVYYCAKPPYSFDSMSESDYWGQGTLVTVSS | 43 |
| | Light chain | QSVLTQPPSVSAAPGQKVTISCSGSGSNVENNYVSWYQQLPGTA PKLLIYENNNRPSGIPDRFSGSKSGTSATLGITGLQTGDEADYYCG | 84 |
| | | VWDDSLNHVIFGGGTKLTVLG | |
| 40 | Heavy chain | QVQLQDSGPGLVKPSETLSLTCTVSGGSISSYDWSWIRQPPGKG LEWIGDIYNSGSTKYNPSLKSRVTISVDTSKNQLSLKLSSVTAADTA VYYCARRGLGNYDILTGYFENAFDIWGQGTTVTVSS | 44 |
| | Light chain | EIVLTQSPSSLSASVGDRVTITCRASQSISSYLNWYQQKPGKAPKL LIYAASSLQSGVPSRFSGSGSGTDFTLTISSLQPEDFATYYCQQSY STPRTFGQGTRLEIKR | 85 |
| 41 | Heavy chain | ETQLVESGGGLVQPGGSLRLSCAASGFTFSSYSMNWVRQAPGK GLEWVSYISSSTSTIYYADSVKGRFTISRDNAKNSLYLQMNSLRAE DTAVYYCARTRLTQLAMIGRGGNAFDIWGQGTMVTVSS | 45 |
| | Light chain | SYELTQPPSVSVAPGKTARITCGGNNIGSKSVHWYQQKPGQAPV LVVYDDSDRPSGIPERFSGSNSGNTATLTISRVEAGDEADYYCQV WDSSSVVFGGGTKLTVLG | 86 |

**[Table 2]**

| Clone | Variable region | Amino acid sequence | SEQ ID NO. |
|---|---|---|---|
| M1 | Heavy chain | EVQLVESGGGLVQPGGSLRLSCAASGFTFSTYYMGWVRQAP GKGLEWVSLISGSGGSTYYDDSVKGRFTISRDNSKNTLYLQM NSLRAEDTAVYYCAKPPYSFDSMSESDYWGQGTLVTVS | 87 |
| | Light chain | QSVLTQPPSVSAAPGQKVTISCSGSGSNVENNYVSWYQQLP GTAPKLLIYENNNRPSGIPDRFSGSKSGTSATLGITGLQTGDE ADYYCGVWDDSLNHVIFGGGTKLTVLG | 92 |
| M2 | Heavy chain | QAYLQQSGAELVRSGASVKLSCTASGFNIKDYYMHWVKQRP EQGLEWIGWIDPENGDTEYAPKFQGKATMTADTSSNTAYLQL SSLTSEDTAVYYCNEDGNYDYWGQGTTLTVSS | 88 |
| | Light chain | DVVVTQSPASLAVSLGQRATISCKASQSVDYAGDSYMNWYQ QKPGQPPKLLIYAASNLESGIPARFSGSGSGTDFTLNIHPVEEE DAATYYCQQSNEDPWTFGGGTKLEIKR | 93 |
| M3 | Heavy chain | EVQLLESGGGLVKPGGSLKLSCAASGFTFSSYDMSWVRQTP EKRLDWVASISSSGGTYYPDSVKGRFTISRDIARNILYLQMNSL RSEDTAMYYCVRGDYWGQGTTLTVSS | 89 |
| | Light chain | DIVMTQSHKFMSASVGDRVNITCKASQDVGTAVAWYQQKPG QSPKLLIYWASTRHTGVPDRFTGSGSGTDFTLTISNVQSEDLA DYFCQQYTRYSNTFGGGTKLEIKR | 94 |
| M4 | Heavy chain | DVMLVESGGDLVKPGGSLKLSCAASGFTFSSRGMSWVRQTP DKRLEWVATISFGDIYTYYPDSVKGRFTISRDNAKNTLYLQMS SLKSEDTAIYYCVRPNDGYFDYWGQGTTLTVSA | 90 |
| | Light chain | DIVMTQSHKFMSTSVGDRVSITCKASQDVSTDVAWYQQKPG | 95 |
| | | QSPKLLIYWASTRHTGVPDRFTGSGSGTDYTLTINSVQAEDLA LYYCQQHYSTPWTFGGGTKLEIKR | |
| M5 | Heavy chain | EVKLVESGGDLVKPGGSLKLSCAASGFTFSSRGMSWVRQTP DKRLEWVATISFGDIYTYYPDSVKGRFTISRDNAKNTLYLQMS SLKSEDTAIYYCARPNDGYFDYWGQGTTLTVSS | 91 |
| | Light chain | DVVMTQTPKFLLVSAGDRVTITCKASQSVSNDVAWYQQKPG QSPKLLIYYASNRYTGVPDRFTGSGYGTDFTLTINSVQAEDLA VYYCQQSYSAPYTFGGGTKLEMK | 96 |

**[Table 3]**

| Clone | Variable region | Amino acid sequence | SEQ ID NO. |
|---|---|---|---|
| 5E9 | Heavy chain | EVQLVESGGGLVQPGGSLRLSCAASGFTFTTYDMGWVRQ APGKGPEWVSLISSGGSNTWYDDSVKGRFTISRDNSKNTL YLQMNSLRAEDTAVYYCAK**EKYPYSDCDY**WGQGTLVTVSS | 97 |
| 6D9 | Heavy chain | EVQLVESGGGLVQPGGSLRLSCAASGFTFTDYDMGWVRQ APGKGPEWVSLISGDSGNTWYDDSVKGRFTISRDNSKNTL YLQMNSLRAEDTAVYYCAK**YRGTTKDY**WGQGTLVTVSS | 98 |
| 6H10 | Heavy chain | EVQLVESGGGLVQPGGSLRLSCAASGFTFTNYYMGWVRQ APGKGPEWVSLISYDGSSTWYDDSVKGRFAISRDNSKNTLY LQMNTLRAEDTAVYYCAK**PDY**WGQGTLVTVSS | 99 |

### Experimental Example 3. Expression and Purification of Antibodies

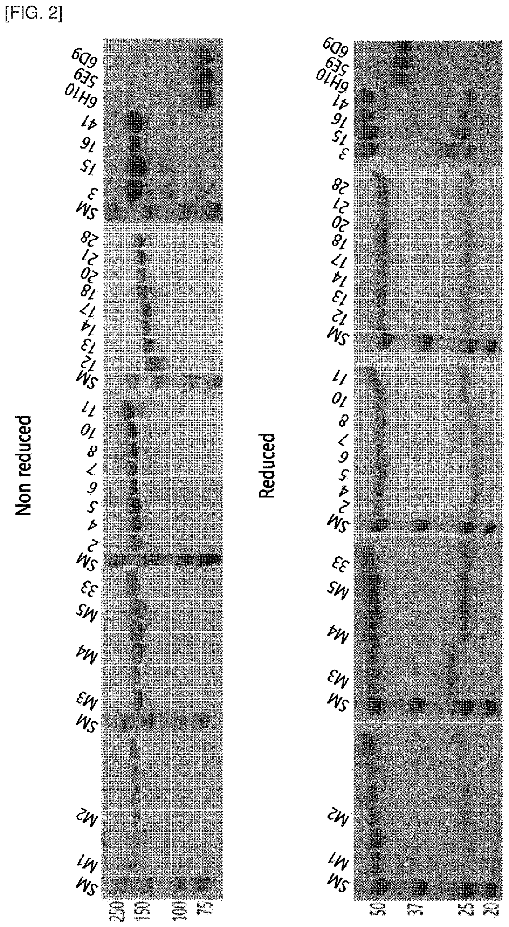

Anti-SARS-CoV-2 scFvs were converted into the human IgG1 format by subcloning into pCIW. Expi293F^{™} cells were prepared in 30 mL of Expi293^{™} expression medium at a concentration of 2.5×10⁶ cells/mL (37°C, 8% CO₂, 125 rpm, viability ≥95%). These cells were transfected with a DNA mixture composed of 30 µg of DNA (pCLW-anti-SARS-CoV-2 heavy chain: 15 µg, pclw-anti-SARS-CoV-2 light chain: 15 µg), OptiPro^{™} SEM medium, and ExpiFectamine^{™} 293 reagent. 0.1 mL of PBS-washed protein A resin was added to the expressed supernatant, followed by incubation overnight at 4°C. After transferring to a column for purification, washing was performed using an IgG binding buffer of 20 times or more of the resin. The bound IgG was eluted using an elution buffer. Concentrations were measured at a wavelength of 280 nm and collected (FIG. 2).

A desalting column was centrifuged at 1000 g for 2 minutes to filter out a preservative solution. After adding PBS buffer according to the capacity of the column, centrifugation was performed at 1000 g for 2 minutes, and column washing was repeated three times. After adding the sample, centrifugation was performed at 1000 g for 2 minutes, and the concentration was re-measured at a wavelength of 280 nm.

### Experimental Example 4. Examination of IgG Cross-Reactivity Through ELISA

ELISA was performed to measure cross-reactivity of 29 clones that were successfully converted to the IgG1 format. An ELISA plate was coated with 50 µL of 3 µg/mL antigen overnight at 4°C. As the antigen, recombinant SARS (1-1190) (Beta Lifescience, BLIT-0210), SARS-CoV-2 (2019-nCoV) Spike Protein (S1+S2 ECD, His tag) (Sino Biological., 40589-V08B1), MERS-CoV Spike Protein (ECD, aa 1-1297, His tag) (Sino Biological., 40069-V08B) were used. Next day, the antigens were removed from the ELISA plate and washed three times with PBS + 0.05% tween 20. Each antigen-coated well was blocked with 200 µL of PBS + 5% BSA for 1 hour at room temperature. After 1 hour, each well was washed three times with PBS + 0.05% tween 20. The concentration of 35 antibodies was diluted to 300 nM and measured. 50 µL of the diluted antibody was added to each well and allowed to bind at room temperature for 1 hour. Each well was washed again three times with PBS + 0.05% tween 20. 50 µL of anti-human Fc-HRP (3000: 1) was added to each well and incubated at room temperature for 1 hour. Each well was washed three times with PBS + 0.05% tween 20. 50 µL of TMB solution was added to each well and incubated for 1 minute to 15 minutes. The reaction was stopped by adding 50 µL of a stop solution to each well. The degree of color development was measured with an ELISA reader.

As a result of the cross-reactivity test for the selected IgG against SARS-CoV-2, SARS-CoV, and MERS-CoV, clones having cross-reactivity toward two or more coronaviruses were identified (Table 4, FIG. 4).

**[Table 4]**

| Clone | SARS-CoV-S1S2 | | SARS-CoV2-S1S2 | | MERS-CoV-S1S2 | |
|---|---|---|---|---|---|---|
| 2 | 2.446 | 2.4908 | 2.027 | 2.027 | 0.0732 | 0.0721 |
| 3 | 2.4794 | 2.5186 | 1.9015 | 1.8947 | 0.07 | 0.0765 |
| 4 | 2.4746 | 2.5391 | 1.9973 | 1.9937 | 0.0769 | 0.0719 |
| 5 | 2.481 | 2.5269 | 1.9471 | 1.9842 | 0.0773 | 0.0743 |
| 6 | 0.0838 | 0.081 | 0.2018 | 0.1948 | 0.0816 | 0.0842 |
| 7 | 0.7374 | 0.7917 | 2.2017 | 2.2618 | 0.0759 | 0.0758 |
| 8 | 1.5787 | 1.6213 | 2.2563 | 2.3055 | 0.0811 | 0.0743 |
| 10 | 1.4732 | 1.6543 | 2.2592 | 2.322 | 0.0824 | 0.0718 |
| 11 | 1.6712 | 1.7502 | 2.189 | 2.243 | 0.0896 | 0.0937 |
| 12 | 1.5002 | 1.5042 | 2.2436 | 2.2271 | 0.0851 | 0.0862 |
| 13 | 1.5442 | 1.5971 | 2.1301 | 2.2009 | 0.0763 | 0.0754 |
| 14 | 1.7089 | 1.6739 | 2.3198 | 2.2674 | 0.0795 | 0.0801 |
| 15 | 1.6942 | 1.6906 | 2.2577 | 2.2371 | 0.0945 | 0.0899 |
| 16 | 1.6185 | 1.6898 | 2.2151 | 2.2353 | 0.072 | 0.0721 |
| 17 | 1.5754 | 1.6017 | 2.1343 | 2.2118 | 0.0837 | 0.087 |
| 18 | 1.6126 | 1.5299 | 2.1413 | 2.2009 | 0.0814 | 0.0772 |
| 20 | 1.2254 | 1.2087 | 2.375 | 2.3156 | 0.1114 | 0.0909 |
| 21 | 1.7254 | 1.7082 | 2.3127 | 2.2976 | 0.0822 | 0.0809 |
| 28 | 0.8419 | 0.8235 | 2.2509 | 2.2324 | 0.0759 | 0.0736 |
| 33 | 1.589 | 1.6232 | 2.081 | 2.128 | 0.1097 | 0.1014 |
| 41 | 1.8776 | 1.9265 | 2.2973 | 2.3247 | 0.0892 | 0.0893 |
| 5E9 | 0.2944 | 0.2779 | 0.8921 | 0.9043 | 0.4069 | 0.3855 |
| 6D9 | 0.3894 | 0.3859 | 0.9972 | 0.9911 | 0.4242 | 0.3948 |
| 6H10 | 0.4795 | 0.4939 | 2.0931 | 2.0575 | 0.5794 | 0.5073 |
| M1 | 0.2098 | 0.1506 | 0.9114 | 0.8545 | 0.3014 | 0.2892 |
| M2 | 0.2735 | 0.2355 | 1.2127 | 1.1994 | 0.5101 | 0.53 |
| M3 | 2.6159 | 2.6175 | 2.2611 | 2.285 | 0.0799 | 0.0732 |
| M4 | 0.1178 | 0.1186 | 1.953 | 1.9824 | 0.0779 | 0.0751 |
| M5 | 0.0773 | 0.0777 | 1.9465 | 1.9842 | 0.0837 | 0.0814 |
| huCCM1 ab | 0.0689 | 0.0709 | 0.1381 | 0.1178 | 0.0698 | 0.0716 |

Specifically, 2, 3, 4, 5, 7, 8, 10, 11, 12, 13, 14, 15, 16, 17, 18, 20, 21, 28, 33, 41, and M3 antibodies (21 kinds) were confirmed to also bind to SARS-CoV1 (cut-off O.D. 0.5 or higher) through the cross-reactivity test by ELISA. In addition, 6H10 and M3 antibodies (two kinds) were confirmed to also bind to MERS-CoV (cut-off O.D. 0.5 or higher). Through this result, it was possible to select clones showing cross-reactivity toward SARS-CoV or MERS-CoV-2 among anti-SARS-CoV-2 antibodies (FIG. 4).

### Experimental Example 5. Preparation of Chimeric Antibodies Against M4 and M5

Humanization was performed on the M4 and M5 clones among the mouse immune clones. The framework region sequences of M4 and M5 antibodies were identified using the mouse germline sequences IGHV5-6 and IGKV6-32, respectively. M4 and M5 clones have the same HCDR sequences and the different LCDR sequences. For the humanization, a general method, CDR grafting, was used. To search for human frameworks having the highest homology to those of M4 and M5, an Igblast search was performed. As a result of the search, they were found to have the highest homology to IGHV3-21 and IGKV1-39, and CDRs of M4 and M5 were grafted to human antibody frameworks, IGHV3-21 and IGKV1-39, respectively. To minimize reductions in stability and affinity due to framework changes after grafting, core residues, which play an important role in determining the stability of the antibody structure and the canonical structure of the CDRs, were identified, and additional mutations were introduced to design variants (Table 5).

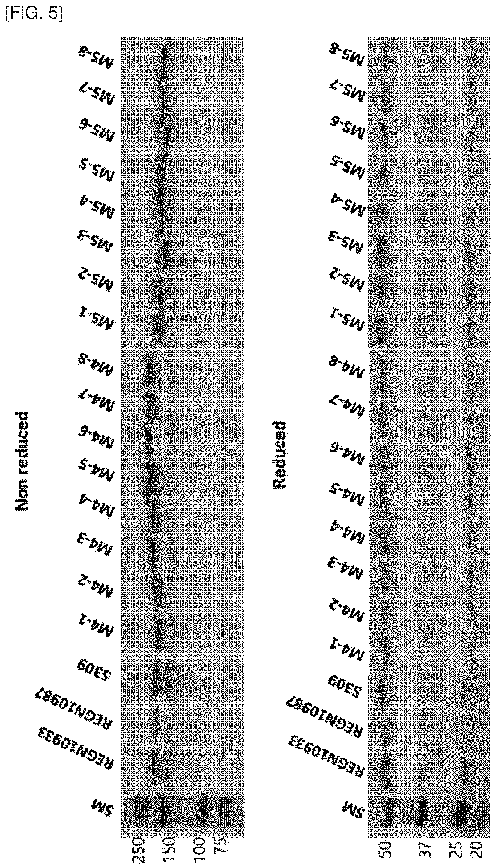

The gene sequences of M4 and M5 antibodies that had been designed were synthesized and used as inserts (SEQ ID NOS: 132 to 138). The synthesized genes were cloned by an infusion cloning method, and culture and purification of the chimeric antibodies were performed as previously described (FIG. 5).

**[Table 5]**

| Clone | Variable region | Amino acid sequence | SEQ ID NO: |
|---|---|---|---|
| M4-1 | Heavy chain (H original) | DVMLVESGGDLVKPGGSLKLSCAASGFTFSSRGMSWVRQTPD KRLEWVATISFGDIYTYYPDSVKGRFTISRDNAKNTLYLQMSSLK SEDTAIYYCVRPNDGYFDYWGQGTTLTVSA | 100 |
| | Light chain (hM4-k2) | DIVMTQSPATLSVSPGERATLSCKASQDVSTDVAWYQQKPGQA PRLLIYWASTRHTGVPDRFSGSGSGTDFTLTISSLQSEDFAVYY CQQHYSTPWTFGGGTKVEIKR | 116 |
| M4-2 | Heavy chain (H original) | DVMLVESGGDLVKPGGSLKLSCAASGFTFSSRGMSWVRQTPD KRLEWVATISFGDIYTYYPDSVKGRFTISRDNAKNTLYLQMSSLK SEDTAIYYCVRPNDGYFDYWGQGTTLTVSA | 101 |
| | Light chain (hM4-k3) | DIVMTQSPATLSVSPGDRVSISCRASQDVSTDVAWYQQKPGQS PKLLIYWASTRHTGVPDRFTGSGSGTDYTLTISSLQPEDFAVYY CQQHYSTPWTFGGGTKVEIKR | 117 |
| M4-3 | Heavy chain (H1) | EVQLVESGGGLVKPGGSLRLSCAASGFTFSSRGMSWVRQAPG KGLEWVSTISFGDIYTYYPDSVKGRFTISRDNAKNSLYLQMNSL RAEDTAVYYCARPNDGYFDYWGQGTLVTVSS | 102 |
| | Light chain (4k original) | DIVMTQSHKFMSTSVGDRVSITCKASQDVSTDVAWYQQKPGQ SPKLLIYWASTRHTGVPDRFTGSGSGTDYTLTINSVQAEDLALY YCQQHYSTPWTFGGGTKLEIKR | 118 |
| M4-4 | Heavy chain (H1) | EVQLVESGGGLVKPGGSLRLSCAASGFTFSSRGMSWVRQAPG KGLEWVSTISFGDIYTYYPDSVKGRFTISRDNAKNSLYLQMNSL RAEDTAVYYCARPNDGYFDYWGQGTLVTVSS | 103 |
| | Light chain (hM4-k2) | DIVMTQSPATLSVSPGERATLSCKASQDVSTDVAWYQQKPGQA PRLLIYWASTRHTGVPDRFSGSGSGTDFTLTISSLQSEDFAVYY CQQHYSTPWTFGGGTKVEIKR | 119 |
| M4-5 | Heavy chain (H1) | EVQLVESGGGLVKPGGSLRLSCAASGFTFSSRGMSWVRQAPG KGLEWVSTISFGDIYTYYPDSVKGRFTISRDNAKNSLYLQMNSL RAEDTAVYYCARPNDGYFDYWGQGTLVTVSS | 104 |
| | Light chain (hM4-k3) | DIVMTQSPATLSVSPGDRVSISCRASQDVSTDVAWYQQKPGQS PKLLIYWASTRHTGVPDRFTGSGSGTDYTLTISSLQPEDFAVYY CQQHYSTPWTFGGGTKVEIKR | 120 |
| M4-6 | Heavy chain (H2) | EVQLVESGGGLVKPGGSLKLSCAASGFTFSSRGMSWVRQAPG KGLEWVATISFGDIYTYYPDSVKGRFTISRDNAKNSLYLQMNSL KAEDTAIYYCARPNDGYFDYWGQGTLVTVSS | 105 |
| | Light chain (4k original) | DIVMTQSHKFMSTSVGDRVSITCKASQDVSTDVAWYQQKPGQ SPKLLIYWASTRHTGVPDRFTGSGSGTDYTLTINSVQAEDLALY YCQQHYSTPWTFGGGTKLEIKR | 121 |
| M4-7 | Heavy chain (H2) | EVQLVESGGGLVKPGGSLKLSCAASGFTFSSRGMSWVRQAPG KGLEWVATISFGDIYTYYPDSVKGRFTISRDNAKNSLYLQMNSL KAEDTAIYYCARPNDGYFDYWGQGTLVTVSS | 106 |
| | Light chain (hM4-k2) | DIVMTQSPATLSVSPGERATLSCKASQDVSTDVAWYQQKPGQA PRLLIYWASTRHTGVPDRFSGSGSGTDFTLTISSLQSEDFAVYY CQQHYSTPWTFGGGTKVEIKR | 122 |
| M4-8 | Heavy chain (H2) | EVQLVESGGGLVKPGGSLKLSCAASGFTFSSRGMSWVRQAPG KGLEWVATISFGDIYTYYPDSVKGRFTISRDNAKNSLYLQMNSL KAEDTAIYYCARPNDGYFDYWGQGTLVTVSS | 107 |
| | Light chain (hM4-k3) | DIVMTQSPATLSVSPGDRVSISCRASQDVSTDVAWYQQKPGQS PKLLIYWASTRHTGVPDRFTGSGSGTDYTLTISSLQPEDFAVYY CQQHYSTPWTFGGGTKVEIKR | 123 |
| M5-1 | Heavy chain (H original) | EVKLVESGGDLVKPGGSLKLSCAASGFTFSSRGMSWVRQTPD KRLEWVATISFGDIYTYYPDSVKGRFTISRDNAKNTLYLQMSSLK SEDTAIYYCARPNDGYFDYWGQGTTLTVSS | 108 |
| | Light chain (hM5-k1) | DIQMTQSPSSLSASVGDRVTITCKASQSVSNDVAWYQQKPGKA PKLLIYYASNRYTGVPSRFSGSGSGTDFTLTISSLQPEDFATYYC QQSYSAPYTFGGGTKVEIKR | 124 |
| M5-2 | Heavy chain (H original) | EVKLVESGGDLVKPGGSLKLSCAASGFTFSSRGMSWVRQTPD KRLEWVATISFGDIYTYYPDSVKGRFTISRDNAKNTLYLQMSSLK SEDTAIYYCARPNDGYFDYWGQGTTLTVSS | 109 |
| | Light chain (hM5-k2) | DVVMTQSPATLSVSPGERATLSCKASQSVSNDVAWYQQKPGQ APRLLIYYASNRYTGVPDRFTGSGSGTDFTLTISSLQSEDFAVYY CQQSYSAPYTFGGGTKVEIKR | 125 |
| M5-3 | Heavy chain (H1) | EVQLVESGGGLVKPGGSLRLSCAASGFTFSSRGMSWVRQAPG KGLEWVSTISFGDIYTYYPDSVKGRFTISRDNAKNSLYLQMNSL RAEDTAVYYCARPNDGYFDYWGQGTLVTVSS | 110 |
| | Light chain (5k original) | DVVMTQTPKFLLVSAGDRVTITCKASQSVSNDVAWYQQKPGQS PKLLIYYASNRYTGVPDRFTGSGYGTDFTLTINSVQAEDLAVYY CQQSYSAPYTFGGGTKLEMK | 126 |
| M5-4 | Heavy chain (H1) | EVQLVESGGGLVKPGGSLRLSCAASGFTFSSRGMSWVRQAPG KGLEWVSTISFGDIYTYYPDSVKGRFTISRDNAKNSLYLQMNSL RAEDTAVYYCARPNDGYFDYWGQGTLVTVSS | 111 |
| | Light chain (hM5-k1) | DIQMTQSPSSLSASVGDRVTITCKASQSVSNDVAWYQQKPGKA PKLLIYYASNRYTGVPSRFSGSGSGTDFTLTISSLQPEDFATYYC QQSYSAPYTFGGGTKVEIKR | 127 |
| M5-5 | Heavy chain (H1) | EVQLVESGGGLVKPGGSLRLSCAASGFTFSSRGMSWVRQAPG KGLEWVSTISFGDIYTYYPDSVKGRFTISRDNAKNSLYLQMNSL RAEDTAVYYCARPNDGYFDYWGQGTLVTVSS | 112 |
| | Light chain (hM5-k2) | DVVMTQSPATLSVSPGERATLSCKASQSVSNDVAWYQQKPGQ APRLLIYYASNRYTGVPDRFTGSGSGTDFTLTISSLQSEDFAVYY CQQSYSAPYTFGGGTKVEIKR | 128 |
| M5-6 | Heavy chain (H2) | EVQLVESGGGLVKPGGSLKLSCAASGFTFSSRGMSWVRQAPG KGLEWVATISFGDIYTYYPDSVKGRFTISRDNAKNSLYLQMNSL KAEDTAIYYCARPNDGYFDYWGQGTLVTVSS | 113 |
| | Light chain (5k original) | DVVMTQTPKFLLVSAGDRVTITCKASQSVSNDVAWYQQKPGQS PKLLIYYASNRYTGVPDRFTGSGYGTDFTLTINSVQAEDLAVYY CQQSYSAPYTFGGGTKLEMK | 129 |
| M5-7 | Heavy chain (H2) | EVQLVESGGGLVKPGGSLKLSCAASGFTFSSRGMSWVRQAPG KGLEWVATISFGDIYTYYPDSVKGRFTISRDNAKNSLYLQMNSL KAEDTAIYYCARPNDGYFDYWGQGTLVTVSS | 114 |
| | Light chain (hM5-k1) | DIQMTQSPSSLSASVGDRVTITCKASQSVSNDVAWYQQKPGKA PKLLIYYASNRYTGVPSRFSGSGSGTDFTLTISSLQPEDFATYYC QQSYSAPYTFGGGTKVEIKR | 130 |
| M5-8 | Heavy chain (H2) | EVQLVESGGGLVKPGGSLKLSCAASGFTFSSRGMSWVRQAPG KGLEWVATISFGDIYTYYPDSVKGRFTISRDNAKNSLYLQMNSL KAEDTAIYYCARPNDGYFDYWGQGTLVTVSS | 115 |
| | Light chain (hM5-k2) | DVVMTQSPATLSVSPGERATLSCKASQSVSNDVAWYQQKPGQ APRLLIYYASNRYTGVPDRFTGSGSGTDFTLTISSLQSEDFAVYY CQQSYSAPYTFGGGTKVEIKR | 131 |

### Experimental Example 6. Assessment of Binding Affinity of Humanized Antibody to Spike Protein

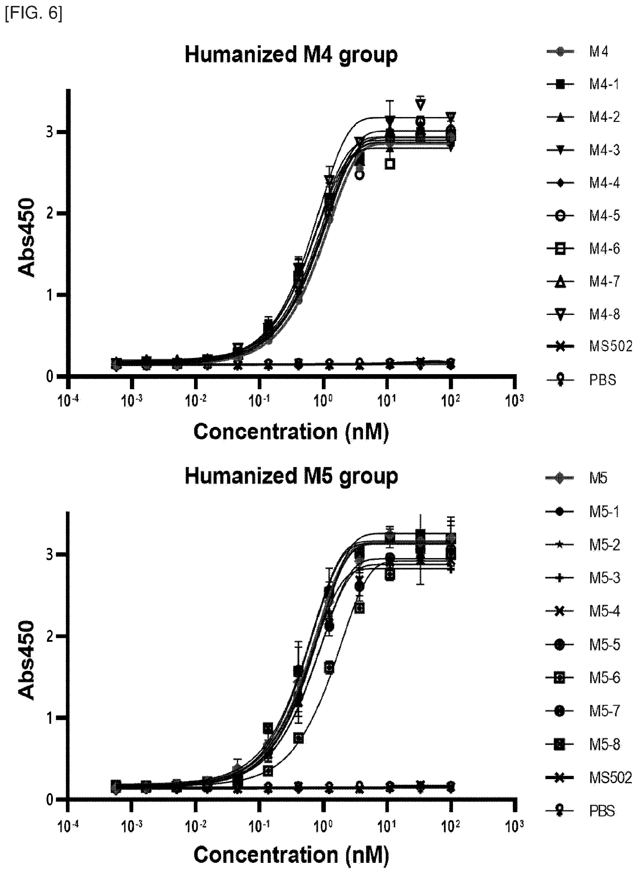

ELISA was performed to examine whether each humanized antibody specifically binds to SARS-CoV-2 spike protein. Each well of an ELISA plate was coated with 100 µL of 3 µg/mL spike protein at 4°C overnight. Next day, the spike proteins were removed from the ELISA plate, and each well was blocked with 200 µL of PBS + 2% BSA at 37°C for 1 hour and washed with PBS + 0.05% tween 20 three times. M4, M5, and each humanized antibody were diluted three-fold with PBS 11 times from the initial concentration (100 nM), and the final concentration was set to 0.5 pM. 100 µL of the diluted antibody solution was added to each well and allowed to bind at 37°C for 1 hour. Then, washing was performed three times with PBS + 0.05% tween 20. 50 µL of HRP conjugated anti-Human IgG-Fc antibody (5000:1) was added to each well, followed by incubation at 37°C for 1 hour. Washing was performed four times with PBS + 0.05% tween 20. 50 µL of TMB solution was added to each well, followed by incubation for 5 minutes. The reaction was stopped by adding 50 µL of a stop solution to each well. As a result of assessing the binding affinity of the humanized clones to the spike protein, it was confirmed that all of them maintained the binding affinity at the levels of M4 and M5 even after humanization (FIG.6).

### Experimental Example 7. Assessment of Binding Affinity of Antibody to RBD and Spike Protein Mutant

ELISA was performed to examine whether or not the humanized antibody M4-4 of M4 and the humanized antibody M5-4 of M5 bind not only to WT of RBD and spike protein but also to various mutants. As the mutants, nine mutants shown in Table 6 below were used.

**[Table 6]**

| Mutant | Company, Product No. | Mutant | Company, Product No. |
|---|---|---|---|
| RBD-V367F | Sino biologics, 40592-V08H1 | RBD-N501Y | Sino biologics, 40592-V08H82 |
| RBD-N439K | Sino biologics, 40592-V08H14 | RBD-A520S | Sino biologics, 40592-V08H20 |
| RBD-S477N | Sino biologics, 40592-V08H46 | RBD-A522S | Sino biologics, 40592-V08H21 |
| RBD-T478I | Sino biologics, 40592-V08H30 | Spike-D614G | Sino biologics, 40589-V08B4 |
| RBD-P479S | Sino biologics, 40592-V08H57 | | |

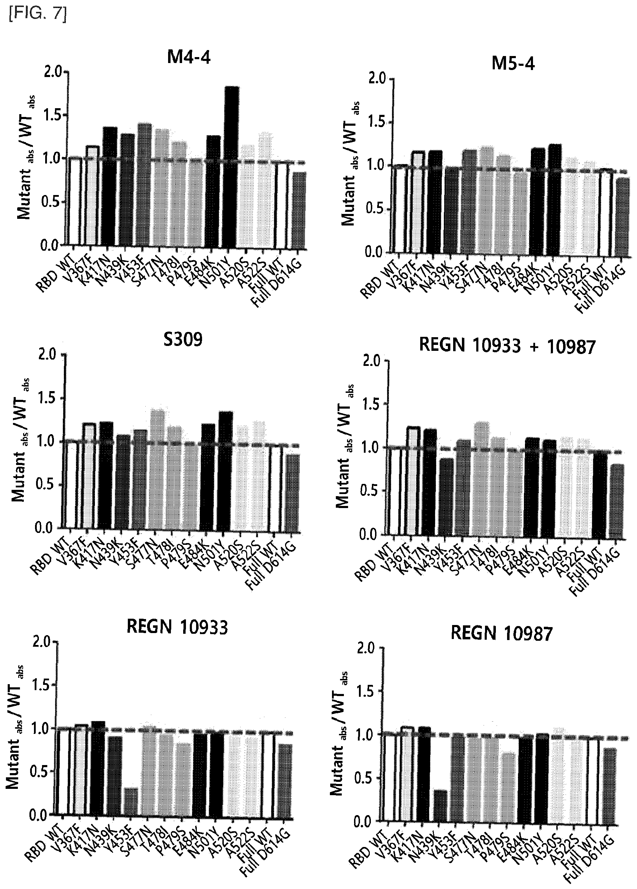

An ELISA plate was coated with 100 µL of 3 µg/mL (0.1 µM) WT and mutants of RBD at 4°C overnight. With regard to the spike protein, 100 µL of 14 µg/mL (0.1 µM) WT and mutants were coated at 4°C overnight. Next day, WT and mutants were removed from the ELISA plate, and each well was blocked with 200 µL of PBS + 2% BSA at 37°C for 1 hour and washed with PBS + 0.05% tween 20 three times. 1 nM of each of the existing antibodies (S309, REGN 10933+10987 combination) and M4, M5, and respective humanized antibodies was added in a volume of 100 µL to each well and allowed to bind at 37°C for 1 hour. Then, washing was performed three times with PBS + 0.05% tween 20. 50 µL of HRP conjugated anti-Human IgG-Fc antibody (5000:1) was added to each well, followed by incubation at 37°C for 1 hour. Washing was performed four times with PBS + 0.05% tween 20. 50 µL of TMB solution was added to each well, followed by incubation for 5 minutes. The reaction was stopped by adding 50 µL of a stop solution to each well. As a result of assessing the binding affinity of M4-4 and M5-4 to the mutants, it was confirmed that all of M4-4 and M5-4 showed the binding affinity for the mutants at the higher levels than WT RBD. With regard to REGN10933 and REGN10987 used as comparative antibodies, when treated alone, their binding affinity for Y453F and N439K mutants was confirmed to reduce by 50% or more (FIG. 7).

### Experimental Example 8. Binding Characterization of Antibodies for S Protein and Variants Thereof

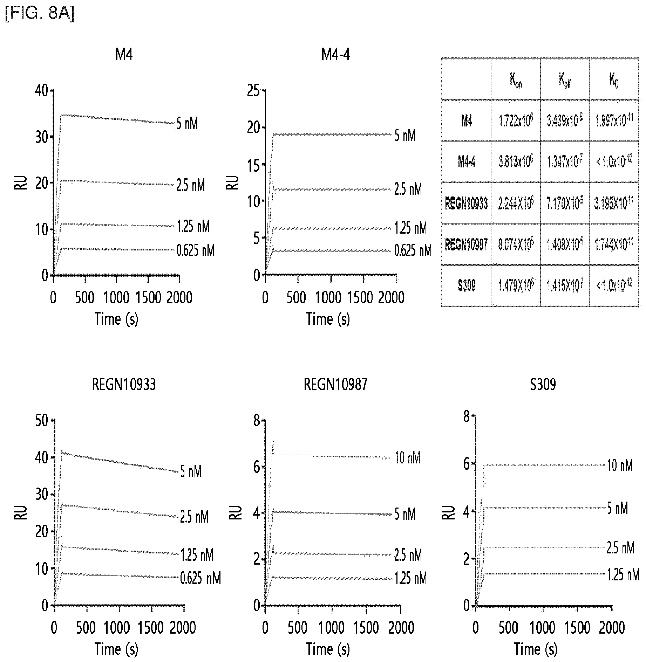

Antigen-antibody binding properties were examined in order to confirm whether or not anti-SARS-CoV-2 spike protein antibodies exhibit different binding properties for the spike protein and variants thereof, alpha (United Kingdom), beta (South Africa), and gamma (Brazil). The kinetics among the binding properties of anti-S protein antibodies were determined using a Biacore T-200 biosensor (Cytiva). The antibodies were diluted with HBS-EP buffer to make up a concentration of 10 µg/mL and captured at a flow rate of 10 µL/min until Rₘₐₓ reached 200 Ru on an SA chip. S protein diluted at a concentration of 0.3125-20 nM in HBS-EP buffer was run on the antibody-captured SA chip at a flow rate of 30 µL/min for 3 minutes for association and 30 minutes for dissociation. Next, 10 mM glycine-HCl (pH 1.5) was reacted for 30 seconds to wash the S protein that was bound to the antibody. A sensorgram of antibodies was analyzed using Biacore evaluation software. As a result of measuring the antigen-antibody binding kinetics, a dissociation constant value enhanced by ten-fold higher was observed in the humanized antibody M4-4, as compared to the mouse antibody M4. This value was confirmed at about ten-fold level, as compared to the comparative antibodies REGN10933 and REGN10987 (FIG. 8A).

Next, the binding ability of the antibodies against the RBD of alpha (United Kingdom), beta (South Africa), and gamma (Brazil) variants were assessed. In this experiment, M4, M4-4, and comparative antibodies (REGN10933, REGN10987, and REGN mix (REGN10933 + 10987, 1:1 mix)) were used. The REGN mix was used to compare whether two antibodies with different RBM epitopes were synergistic in their action against variants when used as a mixture.

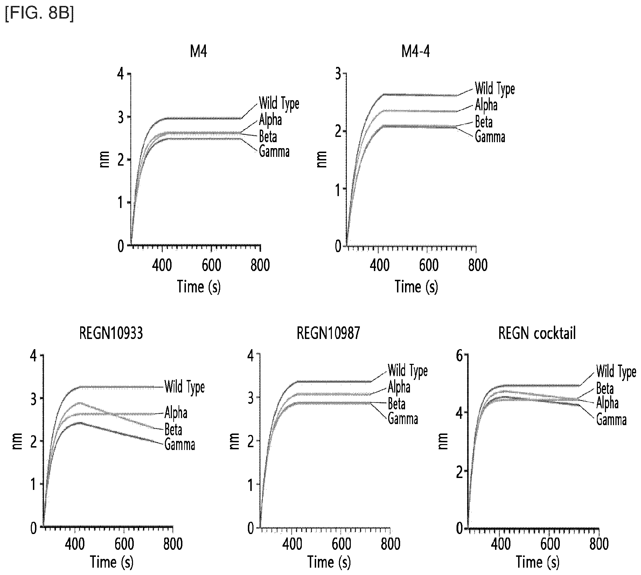

REGN10933, which is an RBM-targeting neutralizing antibody, exhibited a binding affinity for the alpha variant similar to that for the original RBD. However, it was observed that the binding of REGN10933 to beta and gamma variants was significantly reduced. In contrast, M4 and M4-4 showed the similar binding affinity for the existing RBD and all variants (FIG. 8B).

These results suggest that the antibodies of the present invention may retain their ability to bind to newly generated SARS-CoV-2 variants, and they may neutralize various virus variants by having the binding ability thereto.

### Experimental Example 9. Pseudovirus Production

Pseudoviruses bearing the full-length spike protein of SARS-CoV-2 and variants carrying a firefly luciferase reporter gene were produced in Lenti-X 293T cells (Takara). That is, 10 µg of psPAX2 (Addgene), 10 µg of pLVX-Luciferase, and 10 µg of SARS-CoV-2 S (codon-optimized) expression plasmids were co-transfected with polyethylenimine (mass ratio of 1:2) in Lenti-X 293T cells. After 72 hours, the supernatant was centrifuged at 600 xg for 15 minutes, followed by filtration via a 0.45 µm filter and stored at -70°C for further use. Pseudovirus titers were determined by measuring the relative luciferase units.

### Experimental Example 10. Neutralization Assay with SARS-CoV-2 Pseudovirus

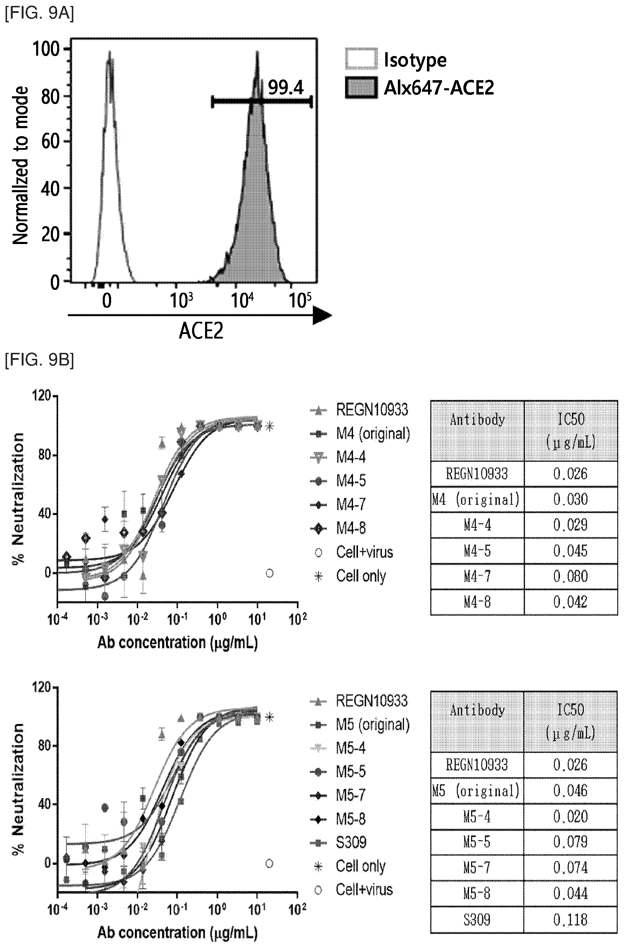

An ACE2-HEK293 stable cell line was maintained in DMEM medium supplemented with 10% fetal bovine serum and an antibiotic-antimycotic cocktail. A 96-well flat bottom plate was coated with 10 µg/mL collagen I and incubated at 37°C in an atmosphere containing 5% CO₂ for 4 hours. ACE2-HEK293 cells were suspended in DMEM and inoculated into a collagen-coated 96-well flat bottom plate at 1×10⁵ cells/well, followed by incubation for 24 hours. Antibodies were serially diluted from 10 µg/mL (66.67 nM) three-fold with DMEM containing 2% FBS and antibiotic-antimycotic cocktail. Antibody dilutions were mixed 1:1 with the pseudovirus and incubated at 37°C in an atmosphere containing 5% CO₂ for 1 hour. The supernatant was removed from ACE2-HEK293 cells and replaced with 50 µL of antibody-pseudovirus mixture. The cells were then incubated at 37°C in an atmosphere containing 5% CO₂ for 1 hour. 50 µL of infectious media was added to the cells, and the cells were further incubated at 37°C in an atmosphere containing 5% CO₂ for 48 hours. The cells were lysed with 5X reporter lysis buffer (Promega), and relative luciferase activity was determined using a luciferase assay system and a luminometer. Relative luciferase units were converted to percent neutralization and plotted with a non-linear regression curve fit in Prism Software (GraphPad, Prism 8.0). As a result, it was confirmed that chimeric antibodies M4 and M5 and humanized antibody candidates M4-4,5,7,8 and M5-4,5,7,8 showed neutralizing potency at a similar level to REGN10933 (FIGS. 9A and 9B).

### Experimental Example 11. Generation of Cell Line Expressing Spike Protein of SARS-CoV-2

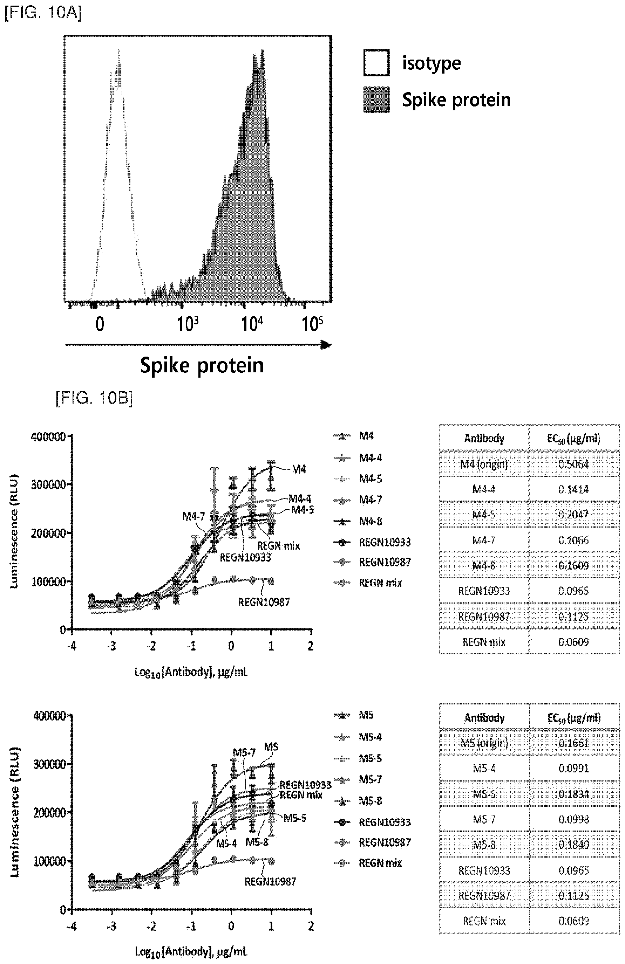

To generate a stable cell line expressing the spike protein of SARS-CoV-2 (HT1080-S), pcDNA3.1-spike cDNA of SARS-CoV-2 was transfected, and the cell lines expressing SARS-CoV-2 S protein were selected with 1.5 mg/mL geneticin. The expression of SARS-CoV-2 S protein was confirmed by flow cytometry (FACS LSR Fortessa, BD Biosciences) using REGN10933, which is an anti-SARS-CoV-2 antibody (FIG. 10A).

### Experimental Example 12. Measurement of Fc-Mediated Effect

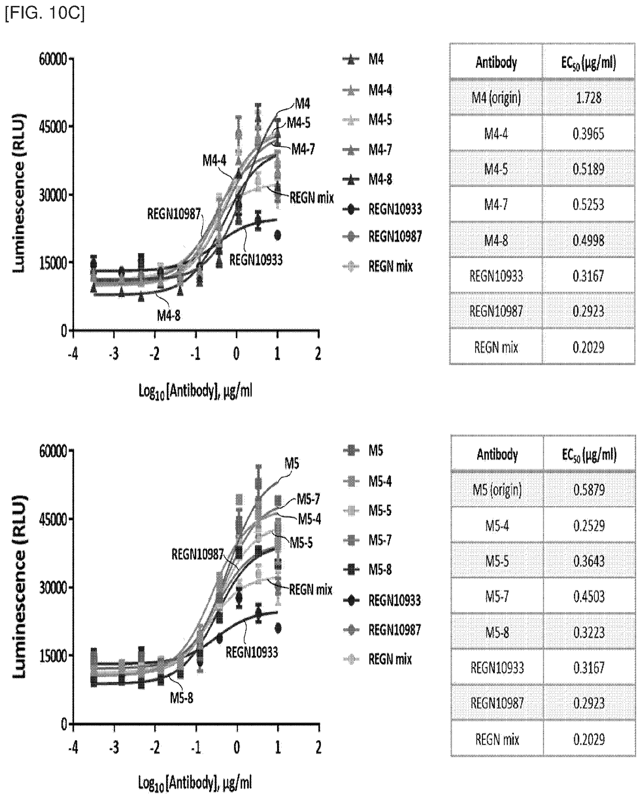

ADCC and ADCP assays were performed to examine whether or not anti-SARS-CoV-2 antibodies exhibit the Fc-mediated antibody-dependent phagocytosis. For the ADCC assay, HT1080-S cells served as target cells and Jurkat-NFAT-Luc/FcγRIIIa served as effector cells. The HT1080-S cells were incubated in an appropriate medium for 18 hours, and ADCC assay buffer was added thereto. Thereafter, the antibodies were diluted and added and allowed to react for 15 minutes. Effector cells were added and incubated for 18 hours. Then, Bio-Glo luciferase assay reagent was added, and luminescence was measured according to the manufacturer's instructions. The measured luminescence values were used to obtain a non-linear regression curve in Prism Software (GraphPad), and EC50 values were obtained. The ADCP assay was performed as described previously. As a result of the assay, chimeric antibodies M4 and M5 and humanized antibody candidates M4-4,5,7,8 and M5-4,5,7,8 showed ADCC efficacy at a similar level to reference antibodies (REGN10987, REGN mix) (FIG. 10B). In addition to ADCC, M4-4 also induced ADCP activity comparable to that of the single Regeneron antibody or Regeneron antibody cocktail (FIG. 10C). Collectively, the data of FIG. 10 show that M4-4 has the potential to induce the Fc-mediated antibody effector functions, ADCC and ADCP, in order to mediate maximal therapeutic efficacy (FIGS. 10B and 10C).

### Experimental Example 13. Measurement of Neutralizing Potency Against SARS-CoV-2 Spike Protein Variants

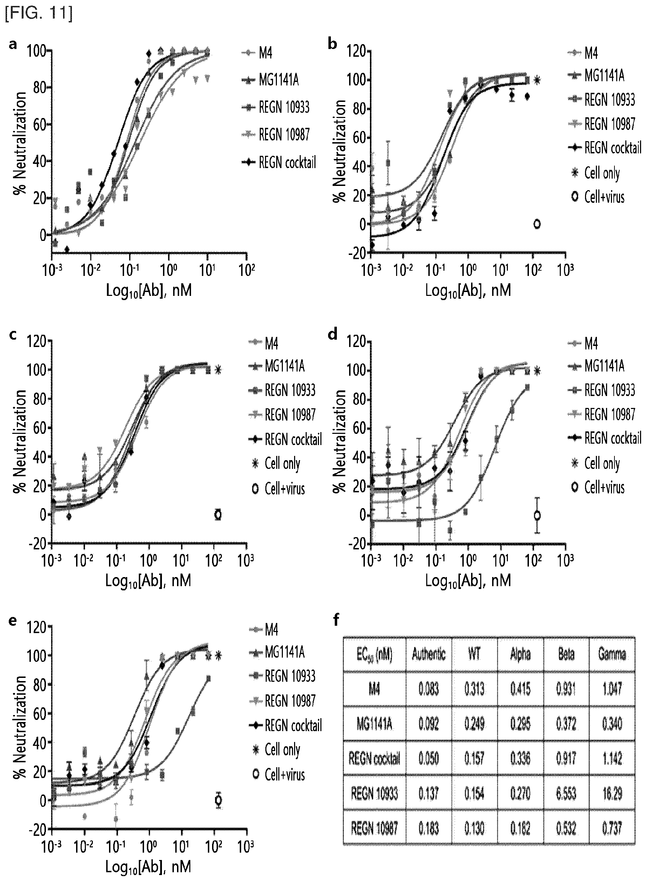

To assess the neutralizing efficacy of M4-4 against SARS-CoV-2 spike variants alpha (United Kingdom), beta (South Africa), and gamma (Brazil), pseudovirus neutralization assays were performed using a lentiviral system expressing each SARS-CoV-2 spike variant. M4 and M4-4 were able to neutralize three variant pseudoviruses with a neutralizing efficacy comparable to that of REGN10987 or Regeneron cocktail (mix). REGN10933 failed to mediate 100% neutralization of beta or gamma variant pseudovirus infection in cells at high concentrations (FIG. 11).

Taken together, the humanized SARS-CoV-2 antibody M4-4 is able to neutralize WT and selected spike-mutant SARS-CoV-2 pseudovirus particles, and its neutralizing efficacy is similar to that of the Regeneron cocktail (FIG. 11).

### Experimental Example 14. Epitope Binning

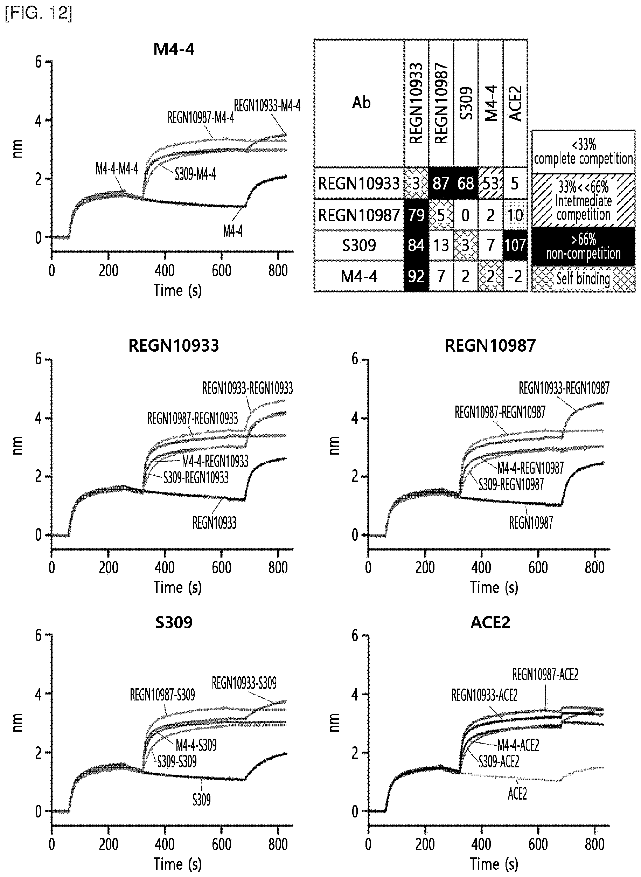

Epitope binning was performed in three steps using the BLI system (Octet Qke): step 1 involved antigen immobilization; step 2 involved 1^{st} antibody binding; and step 3 involved 2^{nd} antibody binding. Between each step, a baseline step with a duration of 60 seconds was set to check the baseline signal. In step 1, 20 µg/mL purified recombinant SARS-CoV-2 spike protein or RBD-His was immobilized in a pre-hydrated anti-penta-His biosensor at 1,000 rpm for 200 seconds. In step 2, 37.5 µg/mL of different 1^{st} binding Abs were bound for 300 seconds. In step 3, one type of the 2^{nd} antibody (18.75 µg/mL) was bound for 150 seconds to check the degree of self-binding and competition. The degree of competition was defined as a percentage. The case in which only the 2^{nd} antibody was bound to the immobilized antigen was defined as 100%. A binding level of less than 33% was defined as complete competition, a level between 33% and 66% was defined as intermediate competition, and a level of 66% or more was defined as non-competition. As a result of binning, it was confirmed that M4-4 competes 100% with the epitope of REG10987 and S309, and partially competes with the epitope of REGN10933. In contrast, REGN10987 and S309 were observed to have a non-competitive epitope with REGN10933 (FIG. 12).

These results suggest that M4-4 is able to bind to a wider range of the epitope region as compared to REGN10933, REGN10987, S309.

In addition, similar experiments were conducted to confirm competitive binding of ACE2 receptor. The difference was that the binding affinity of the recombinant human ACE2 receptor and the recombinant SARS-CoV-2 spike protein RBD was about 10-100 compared with the affinity between mAb and RBD. Thus, the ACE2 receptor was only used for step 3, which involved the 2^{nd} antibody binding. It was observed that M4-4 exhibited 100% competition with the ACE2 receptor, indicating that M4-4 is able to 100% inhibit the binding of antigen to the ACE2 receptor (FIG. 12).

### Experimental Example 15. Simulation Docking

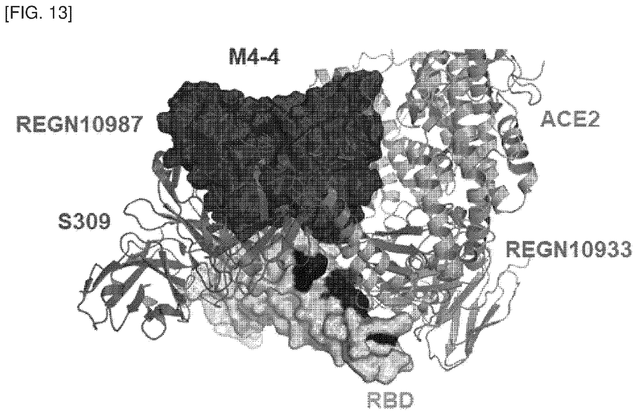

BioLuminate within Schrodinger Suite was used for scFv homology modeling of M4-4 using the structure of anti-PD1 (PDB code: 6JJP) and anti-SIRPα antibodies (PDB code: 6 NMR) as templates for heavy and light chains, respectively. Docking of scFv structures of M4-4 to RBD (PDB code: 6M0J) was performed using PIPER in BioLuminate56. The distances between Lys440 and Thr500 of RBD and CDR loops were constrained between 2 Å and 10 Å based on the results of epitope binning. BioLuminate suggested 30 best complexes with 70,000 possible protein-protein configurations. The final complexes for M4-4 were selected according to the cluster size and agreement with the epitope binning results. Then, energy minimization was performed. The binding site of M4-4 to RBD, predicted by the result of simulation docking, was a site capable of interfering with the binding of REGN10933, REGN10987, S309, and ACE2 receptor. This is consistent with the binning results, and it was possible to predict the epitope of M4-4 (FIG. 13).

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

### Effect of the invention

The antibody of the present invention may be usefully used in the detection and diagnosis of coronavirus, and also usefully used in the prevention and treatment of coronavirus infectious diseases, owing to its neutralization potency against coronavirus.

## Claims

1. An antibody specifically binding to a coronavirus spike protein or an antigen binding fragment thereof.

2. The antibody or antigen binding fragment thereof of claim 1, wherein the antibody or antigen binding fragment thereof includes any one or more heavy chain variable regions selected from the group including any one sequence of SEQ ID NOS: 5 to 45, SEQ ID NOS: 87 to 91, and SEQ ID NOS: 97 to 115; and any one or more light chain variable regions selected from the group including any one sequence of SEQ ID NOS: 46 to 86, SEQ ID NOS: 92 to 96, and SEQ ID NOS: 116 to 131.

3. The antibody or antigen binding fragment thereof of claim 1, wherein the antibody or antigen binding fragment thereof includes any one or more heavy chain variable regions selected from the group including any one sequence of SEQ ID NOS: 87 to 91 and SEQ ID NOS: 100 to 115; and any one or more light chain variable regions selected from the group including any one sequence of SEQ ID NOS: 92 to 96 and SEQ ID NOS: 116 to 131.

4. The antibody or antigen binding fragment thereof of claim 1, wherein the coronavirus is SARS-CoV, SARS-CoV-2, or MERS-CoV.

5. The antibody or antigen binding fragment thereof of claim 1, wherein the antibody or antigen binding fragment thereof has cross-reactivity with two or more kinds of coronaviruses.

6. The antibody or antigen binding fragment thereof of claim 5, wherein the antibody or antigen binding fragment thereof having cross-reactivity with two or more kinds of coronaviruses includes one or more heavy chain variable regions selected from the group including any one sequence of SEQ ID NOS: 6 to 12, SEQ ID NOS: 14 to 22, SEQ ID NOS: 24, 25, 32, 37, and 45, SEQ ID NOS: 87 to 91, and SEQ ID NOS: 97 to 99; and one or more light chain variable regions selected from the group including any one sequence of SEQ ID NOS: 47 to 53, SEQ ID NOS: 55 to 63, SEQ ID NOS: 65, 66, 73, 78, and 86, and SEQ ID NOS: 92 to 96.

7. The antibody or antigen binding fragment thereof of claim 1, wherein the antibody or antigen binding fragment thereof includes a heavy chain variable region including a heavy chain CDR1 of SEQ ID NO: 139; a heavy chain CDR2 of SEQ ID NO: 140; and a heavy chain CDR3 of SEQ ID NO: 141, and a light chain variable region including a light chain CDR1 of SEQ ID NO: 142 or 145; a light chain CDR2 of SEQ ID NO: 143 or 146; and a light chain CDR3 of SEQ ID NO: 144 or 147.

8. The antibody or antigen binding fragment thereof of claim 1, wherein the heavy chain variable region consists of an amino acid sequence of SEQ ID NO: 103; and
the light chain variable region consists of an amino acid sequence of SEQ ID NO: 119.

9. A polynucleotide encoding the antibody or antigen binding fragment thereof of any one of claims 1 to 8.

10. An expression vector comprising the polynucleotide of claim 9.

11. A transformant introduced with the expression vector of claim 10.

12. A pharmaceutical composition for preventing or treating coronavirus infection, the pharmaceutical composition comprising the antibody or antigen binding fragment thereof of any one of claims 1 to 8.

13. A method of preventing or treating coronavirus infection, the method comprising the step of administering the antibody or antigen binding fragment thereof of any one of claims 1 to 8 to an individual excluding humans.

14. An antibody-drug conjugate, wherein a drug is conjugated to the antibody or antigen binding fragment thereof of any one of claims 1 to 8.

15. A composition for diagnosing coronavirus infection, the composition comprising the antibody or antigen binding fragment thereof of any one of claims 1 to 8.

16. A kit for diagnosing coronavirus infection, the kit comprising the composition of claim 15.

17. A method of providing information for diagnosing coronavirus infection, the method comprising the step of detecting coronavirus present in a biological sample separated from an individual suspected of having coronavirus infection using the antibody or antigen binding fragment thereof of any one of claims 1 to 8; or a composition for diagnosing the infection, the composition including the antibody or antigen binding fragment thereof; or a kit including the composition.
